# EUROPEAN PATENT APPLICATION

(11) **EP 2 383 813 A1**
(43) Date of publication of application: **02.11.2011**
(21) Application number: 10733459.1
(22) Date of filing: 19.01.2010
(51) Int. Cl.: H01L 51/50, C07D 209/14, C07D 213/22, C07D 235/08, C07D 333/18, C07D 333/54, C07D 471/04, C07F 5/02, C09K 11/06

(54) **LIGHT-EMITTING ELEMENT MATERIAL AND LIGHT-EMITTING ELEMENT**

(30) Priority: 23.01.2009 JP 2009012636
(71) Applicant: Toray Industries, Inc., Tokyo, 103-8666 (JP)
(72) Inventor: SUGIMOTO, Kazunori, Otsu-shi Shiga 520-8558 (JP); TOMINAGA, Tsuyoshi, Otsu-shi Shiga 520-8558 (JP)
(74) Representative: Hager, Thomas Johannes
(86) International application number: PCT/JP2010/050541
(87) International publication number: WO 2010/084852

(57) **Abstract**

Disclosed is a light-emitting element which has high luminous efficiency and excellent durability. Specifically disclosed is a light-emitting which comprises at least a light-emitting layer between a positive electrode and a negative electrode, and emits light by electrical energy. The light-emitting element is **characterized by** containing a naphthacene compound that has a specific structure and organic fluorescent substance that has a fluorescence peak wavelength of not less than 500nm but not more than 690 nm.

## Description

### TECHNICAL FIELD

The present invention relates to a naphthacene compound useful as a fluorescent dye and a charge transport material, and a light-emitting element using the same, which can be used in the fields of display elements, flat panel displays, backlights, illumination, interior materials, signs, signboards, electrophotographic machines, light signal generators, etc.

### BACKGROUND ART

Research on an organic thin-film light-emitting element in which the electrons injected from a cathode and the holes injected from an anode are coupled again to emit light in an organic light emitter held between the cathode and the anode, is actively -conducted in recent years. The light-emitting element attracts attention, since it is characterized by the thinness of the element, highly bright light emission at a low drive voltage, and multi-color light emission available by selecting light-emitting materials. Since C. W. Tang et al. of Kodak demonstrated that an organic thin-film light-emitting element emitted highly bright light, many research organizations are pursuing the research. The typical configuration of the organic thin-film light-emitting element presented by the research group of Kodak was such that a hole-transportable diamine compound, tris(8-quinolinolate)aluminum (III) as a light-emitting layer and Mg:Ag as a cathode were formed one after another on an ITO glass substrate, and at a drive voltage of approximate. 10 V, green light emission of 1,000 cd/m² was possible (see Non-Patent Document 1). Further, since organic thin-film light-emitting elements can emit diverse light colors by using various fluorescent materials in the light-emitting layers, active studies are being made for practical application to displays, etc. Especially studies on light-emitting materials of three primary colors of red, green and blue are most active, and earnest studies are being made for enhancing properties.

One of the largest problems of organic thin-film light-emitting elements is to enhance the durability and light emission efficiency of the elements. Especially with regard to red light-emitting elements, there are few redlight-emitting materials which can provide elements with excellent durability and high reliability. For example, though light-emitting elements using naphthacene compounds (see Patent Documents 1 to 3) are disclosed, they are insufficient in durability. Further, as a means for obtaining a highly efficient light-emitting element, a method of forming a light-emitting layer by doping a host material with several percent of a dopant material (fluorescent material) is known. The host material is required to have high carrier mobility, uniform film formability, etc. , and the dopant material is required to have high fluorescence quantum yield, uniform dispersibility, etc. For example, red materials include aminostyryl compounds (see Patent Document 4), diketopyrrolopyrrole derivatives and pyrromethene compounds (see Patent Document 5), naphthacene derivatives and pyrromethene compounds (see Patent Documents 6 and 7), cumarine compounds and dicyanomethylenepyran compounds (see Patent Document 8) , etc. , but there was no compound sufficient in both light emission efficiency and durability.

### PRIOR ART DOCUMENTS

### PATENT DOCUMENTS

Patent Document 1: Japanese Patent No. 3196230
Patent Document 2: JP 6-228555 A
Patent Document 3: WO 2007/097178
Patent Document 4: JP 2002-134276 A
Patent Document 5: JP 2003-86379 A
Patent Document 6: WO 2008/047744
Patent Document 7: JP 2008-159777 A
Patent Document 8: JP 5-202356 A

### NON-PATENT DOCUMENT

Non-Patent Document 1: Applied Physics Letters, USA, 1987, Vol. 51, No. 12, pages 913 to 915

### SUMMARY OF THE INVENTION

### PROBLEMS TO BE SOLVED BY THE INVENTION

As described above, organic thin-film light-emitting elements with high light emission efficiency and excellent durability have not been provided. The object of this invention is to provide a light-emitting element material that can be processed into a light-emitting element with high light emission efficiency and excellent durability, and also to provide a light-emitting element using the light-emitting element material.

### MEANS FOR SOLVING THE PROBLEMS

The light-emitting element material of this invention comprises a naphthacene compound represented by general formula (1).

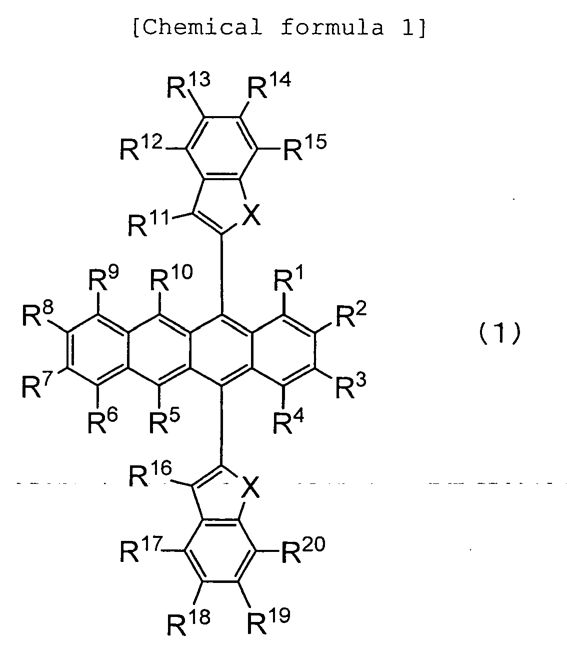

Further, the light-emitting element of this invention is a light-emitting element capable of emitting light with electric energy, in which at least an organic layer exists between an anode and a cathode, wherein any one of the layers existing between the anode and the cathode contains the abovementioned light-emitting element material.

### EFFECTS OF THE INVENTION

This invention can provide a light-emitting element with high light emission efficiency and excellent durability.

### MODES FOR CARRYING OUT THE INVENTION

The naphthacene compound represented by the general formula (1) in this invention is explained below in detail.

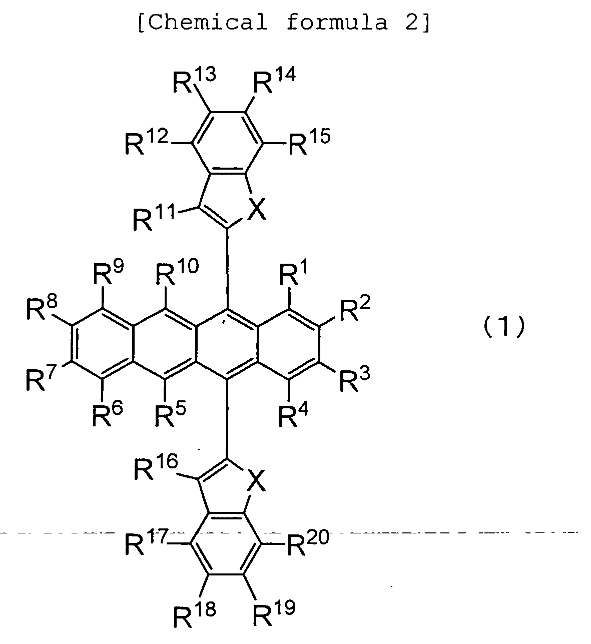

(where R¹ to R²⁰ can be identical to or different from each other, and are respectively selected from hydrogen, alkyl group, cycloalkyl group, heterocyclic ring group, alkenyl group, cycloalkenyl group, alkynyl group, alkoxy group, alkylthio group, aryl ether group, aryl thioether group, aryl group, heteroaryl group, halogen, carbonyl group, carboxyl group, oxycarbonyl group, carbamoyl group, amino group and silyl group; each pair of substituent groups adjacent to each other denoted by R¹² to R¹⁵ and R¹⁷ to R²⁰ can form a ring; X is selected from an oxygen atom, sulfur atom and -NR²¹-; and R²¹ is selected from hydrogen, alkyl group, cycloalkyl group, heterocyclic ring group, aryl group and heteroaryl group.)
Among these substituent groups, an alkyl group refers to a saturated aliphatic hydrocarbon group such as methyl group, ethyl group, propyl group or butyl group. The alkyl group may be non-substituted or substituted. In the case where the alkyl group is substituted, the substituent group is not especially limited and can be, for example, an alkyl group, aryl group or heteroaryl group, etc., and this applies also in the following explanation. Further, the number of carbon atoms of the alkyl group is not especially limited, but in view of easy availability and cost, usually the number of carbon atoms is in a rage from 1 to 20. A more preferred range- is 1 to 8.

Further, a cycloalkyl group refers to a saturated alicyclic hydrocarbon group such as cyclopropyl group, cyclohexyl group, norbornyl group or adamantyl group. The cycloalkyl group may be non-substituted or substituted. The number of carbon atoms of the cycloalkyl group is not especially limited, but is usually in a range from 3 to 20.

Furthermore, a heterocyclic ring group refers to a group comprising an aliphatic ring having an atom other than carbon in the ring such as pyran ring, piperidine ring or cyclic amide. The heterocyclic ring group may be non-substituted or substituted. The number of carbon atoms of the heterocyclic ring group is not especially limited, but is usually in a range from 2 to 20.

Moreover, an alkenyl group refers to an unsaturated aliphatic hydrocarbon group containing a double bond such as vinyl group, allyl group or butadienyl group. The alkenyl group may be non-substituted or substituted. The number of carbon atoms of the alkenyl group is not especially limited, but is usually in a range from 2 to 20.

Further, a cycloalkenyl group refers to an unsaturated alicyclic hydrocarbon group containing a double bond such as cyclopentenyl group, cyclopentadienyl group or cyclohexenyl group. The cycloalkenyl group may be non-substituted or substituted.

Furthermore, an alkynyl group refers to an unsaturated aliphatic hydrocarbon group containing a triple bond such as ethynyl group. The alkynyl group may be non-substituted or substituted. The number of carbon atoms of the alkynyl group is not especially limited, but is usually in a range from 2 to 20.

Moreover, an alkoxy group refers to an aliphatic hydrocarbon group with an ether linkage interposed such as methoxy group. The alkoxy group may be non-substituted or substituted. The number of carbon atoms of the alkoxy group is not especially limited, but is usually in a range from 1 to 20.

Further, an alkylthio group refers to a group in which the oxygen atom of the ether linkage of an alkoxy group is substituted by a sulfur atom.

Furthermore, an aryl ether group refers to an aromatic hydrocarbon group with an ether linkage interposed such as phenoxy group. The aryl ether group may be non-substituted or substituted. The number of carbon atoms of the aryl ether group is not especially limited, but is usually in a range from 6 to 40.

Moreover, an aryl thioether group refers to a group in which the oxygen atom of the ether linkage of an aryl ether group is substituted by a sulfur atom.

Further, an aryl group refers to an aromatic hydrocarbon group such as phenyl group, naphthyl group, biphenyl group, phenanthryl group, terphenyl group, pyrenyl group, anthracenyl group or fluorenyl group. The aryl group may be non-substituted or substituted. The number of carbon atoms of the aryl group is not especially limited, but is usually in a range from 6 to 40.

Furthermore, a heteroaryl group refers to an aromatic group having an atom other than carbon in the ring such as furanyl group, thiophenyl group, oxazolyl group, pyridyl group or quinolinyl group, and the heteroaryl group may be non-substituted or substituted. The number of carbon atoms of the heteroaryl group is not especially limited, but is usually in a range from 2 to 30.

An amino group may be non-substituted or substituted, and the substituent group can be, for example, an alkyl group, cycloalkyl group, aryl group or heteroaryl group, and these substituent groups may also be further substituted.

A silyl group refers to a functional group having a linkage to a silicon atom such as trimethylsilyl group, and the silyl group is not required to have a substituent group but may also have a substituent group. The number of carbon atoms of the silyl group is not especially limited, but is usually in a range from 3 to 20. Further, the number of silicon atoms is usually in a range from 1 to 6.

The ring structure that may be formed between adjacent groups is explained below in reference to the aforementioned general formula (1). The ring structure means such a structure in which arbitrary adjacent two substituent groups selected from R¹² to R¹⁵ and R¹⁷ to R²⁰ (for example, R¹² and R¹³) are coupled with each other to form a conjugated or non-conjugated condensed ring. The condensed ring may contain one or more types of nitrogen, oxygen and sulfur atoms in the annular structure, or can also be condensed with another ring. It is preferred that the atoms constituting the condensed ring are carbon atoms and hydrogen atoms only, since excellent heat resistance can be obtained.

The naphthacene compound represented by the general formula (1) has two bicyclic benzohetero rings (benzofuran rings, benzothiophene rings or indole rings) at specific binding positions (5-position and 12-position) of the naphthacene skeleton in the molecule. This configuration allows both high carrier mobility and heat resistance to be achieved, and allows the light emission efficiency and durability of the element to be enhanced.

It is preferred that R¹¹ and R¹⁶ denote respectively an aryl group or heteroaryl group for such reasons that the intermolecular interaction between naphthacene skeletons can be inhibited for allowing high light emission efficiency to be achieved and for allowing a thin film to be formed stably. Above all, it is more preferred that R¹¹ and R¹⁶ denote respectively an aryl group or heteroaryl group with 4 to 14 carbon atoms, since raw material acquisition and synthesis process are easy to allow cost reduction.

Examples of the aryl group or heteroaryl group with 4 to 14 carbon atoms include a phenyl group, naphthyl group, phenanthryl group, anthracenyl group, fluorenyl group, furanyl group, thiophenyl group, pyrrolyl group, benzofuranyl group, benzothiophenyl group, indolyl group, benzoxazolyl group, benzothiazolyl group, benzimidazolyl group, pyridyl group, quinolinyl group, quinoxanyl group, carbazolyl group and phenanthrolyl group. Among them, in view of high light emission efficiency, thin film stability and easiness of raw material acquisition and synthesis process, preferred are a phenyl group, naphthyl group, phenanthryl group, fluorenyl group, benzofuranyl group, benzothiophenyl group, pyridyl group, quinolinyl group and quinoxanyl group.

Meanwhile, the abovementioned aryl group and heteroaryl group can also further have a substituent group. In this case, the examples of the substituent group include alkyl groups such as methyl group, ethyl group, propyl group and t-butyl group, alkoxy groups such as methoxy group and ethoxy group, aryl ether groups such as phenoxy group, aryl groups such as phenyl group, naphthyl group and biphenyl group, heteroaryl groups such as pyridyl group, quinolinyl group, benzofuranyl group and dibenzofuranyl group. Above all, in view of easiness of raw material acquisition and synthesis process, a methyl group, t-butyl group and phenyl group are especially preferred.

Further, it is preferred that X of general formula (1) denotes an oxygen atom, since higher light emission efficiency can be achieved.

In order to synthesize the naphthacene compound represented by general formula (1), a publicly known method can be used. The method for introducing bicyclic benzohetero rings into the naphthacene skeleton can be, for example, a method of using a coupling reaction by a naphthoquinone derivative and a bicyclic benzohetero ring metal reagent, or a method of using a coupling reaction between a hydrogenated naphthacene derivative and a bicyclic benzohetero ring boric acid in the presence of a palladium or nickel catalyst, etc., though not limited to these methods.

Examples of the naphthacene compound having a group containing bicyclic benzohetero rings represented by the abovementioned general formula (1) include the following.

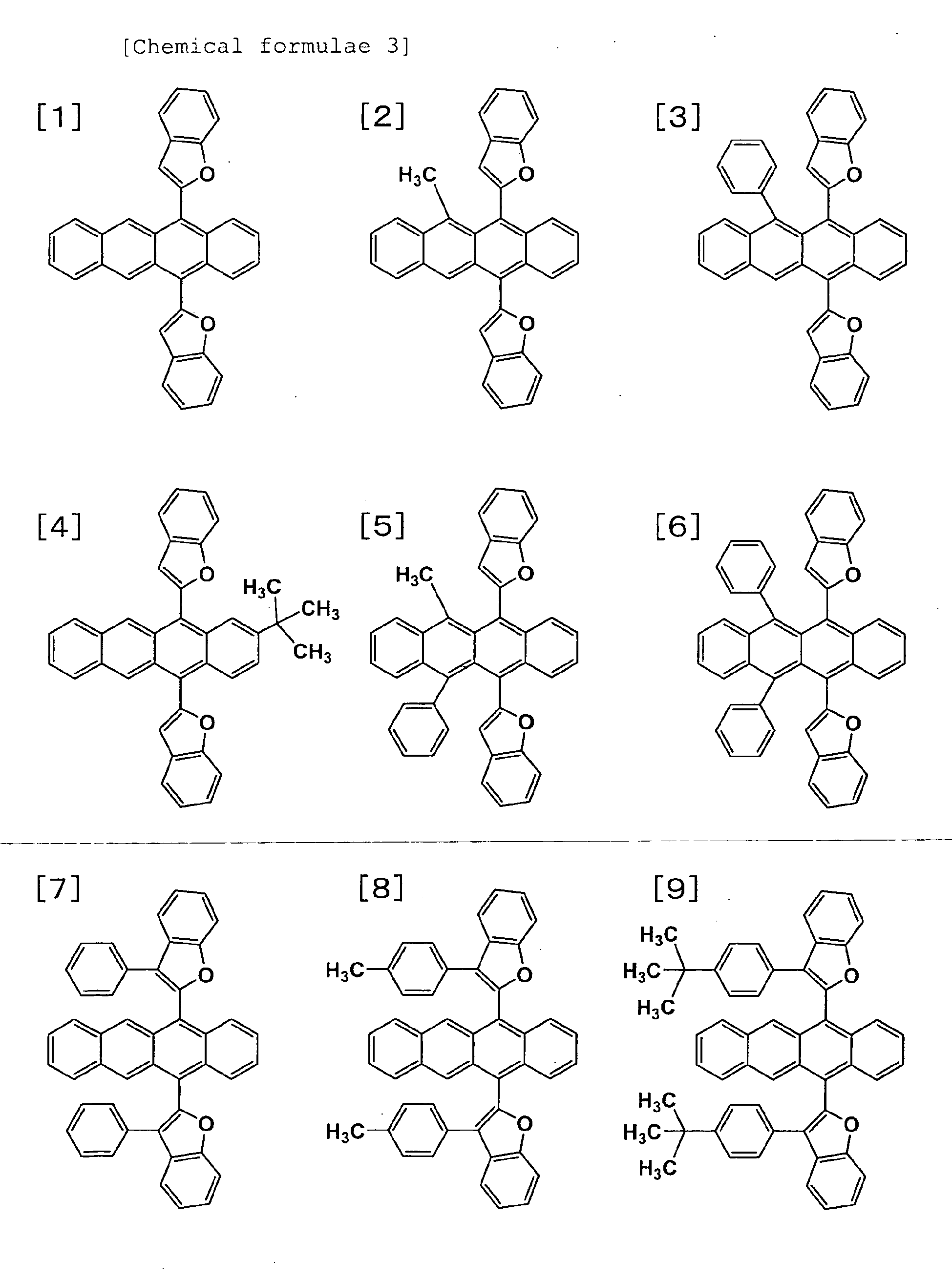

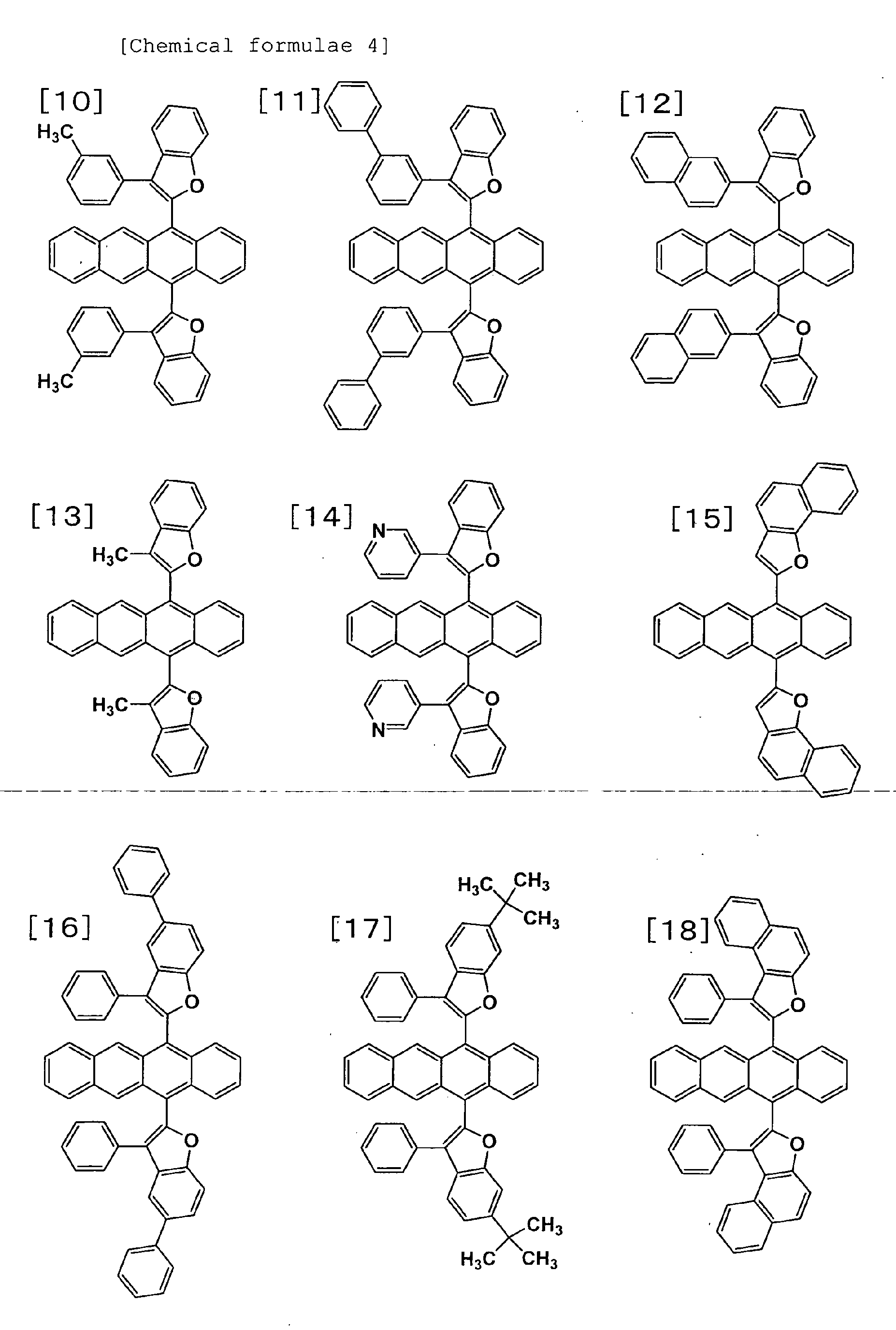

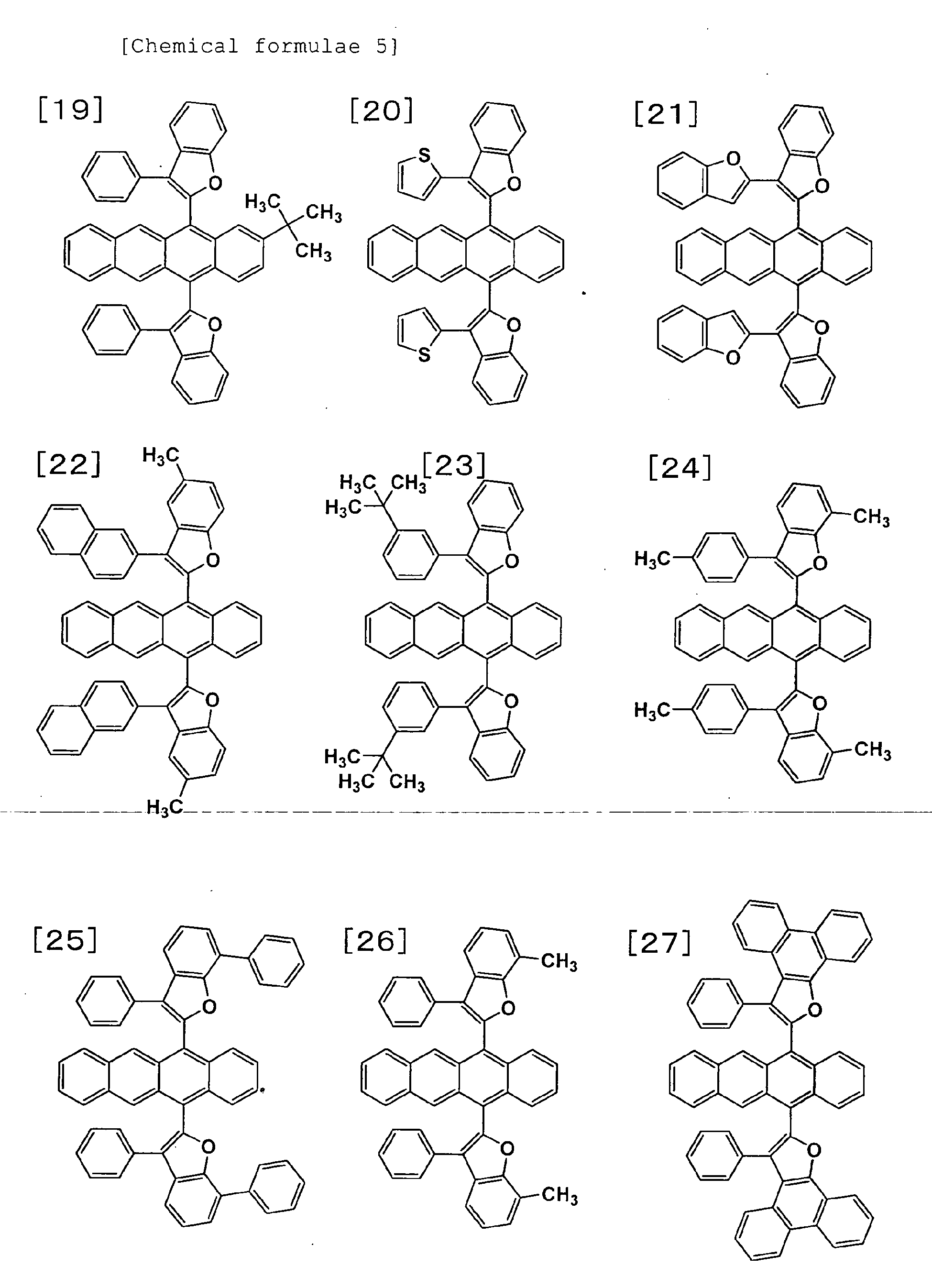

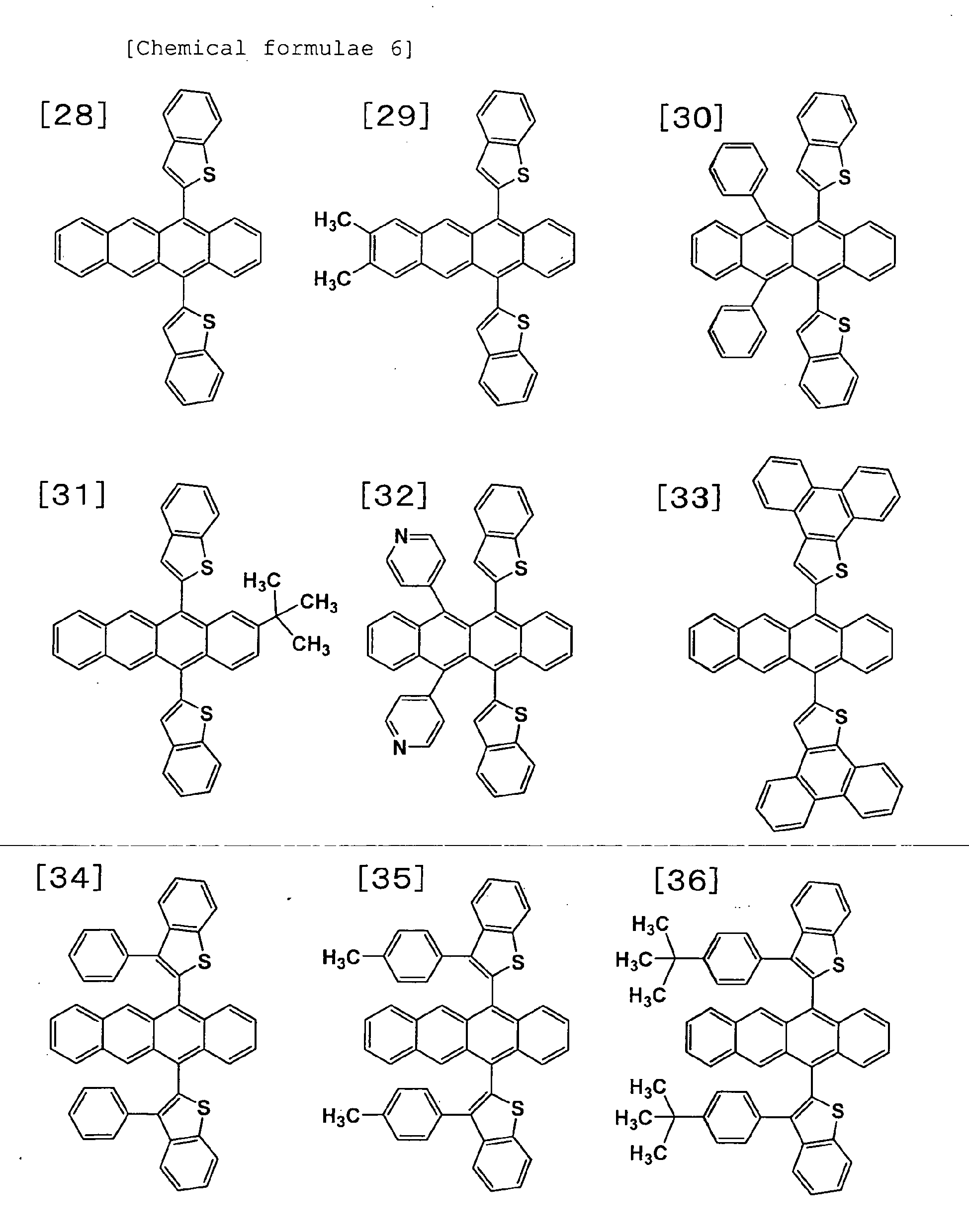

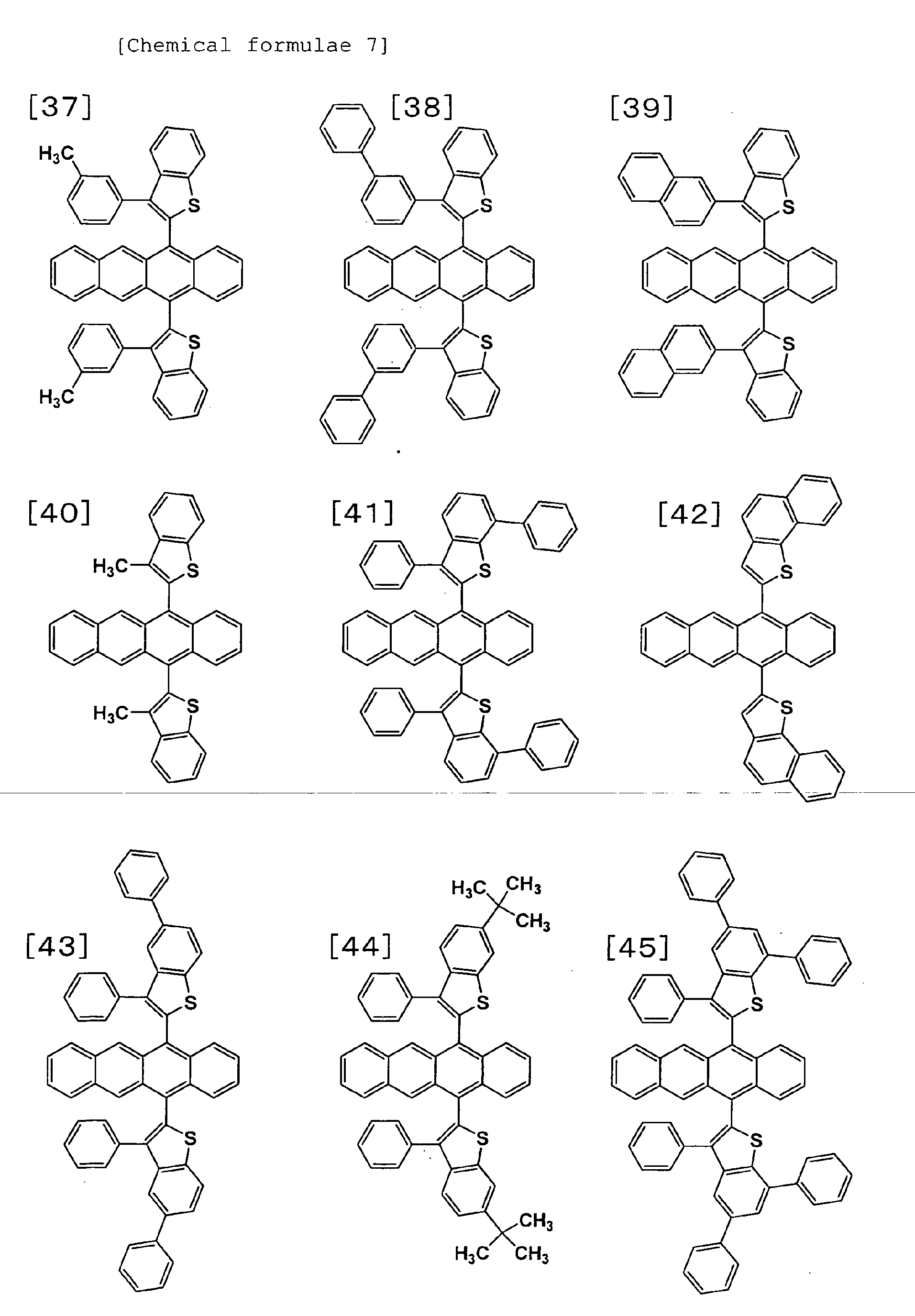

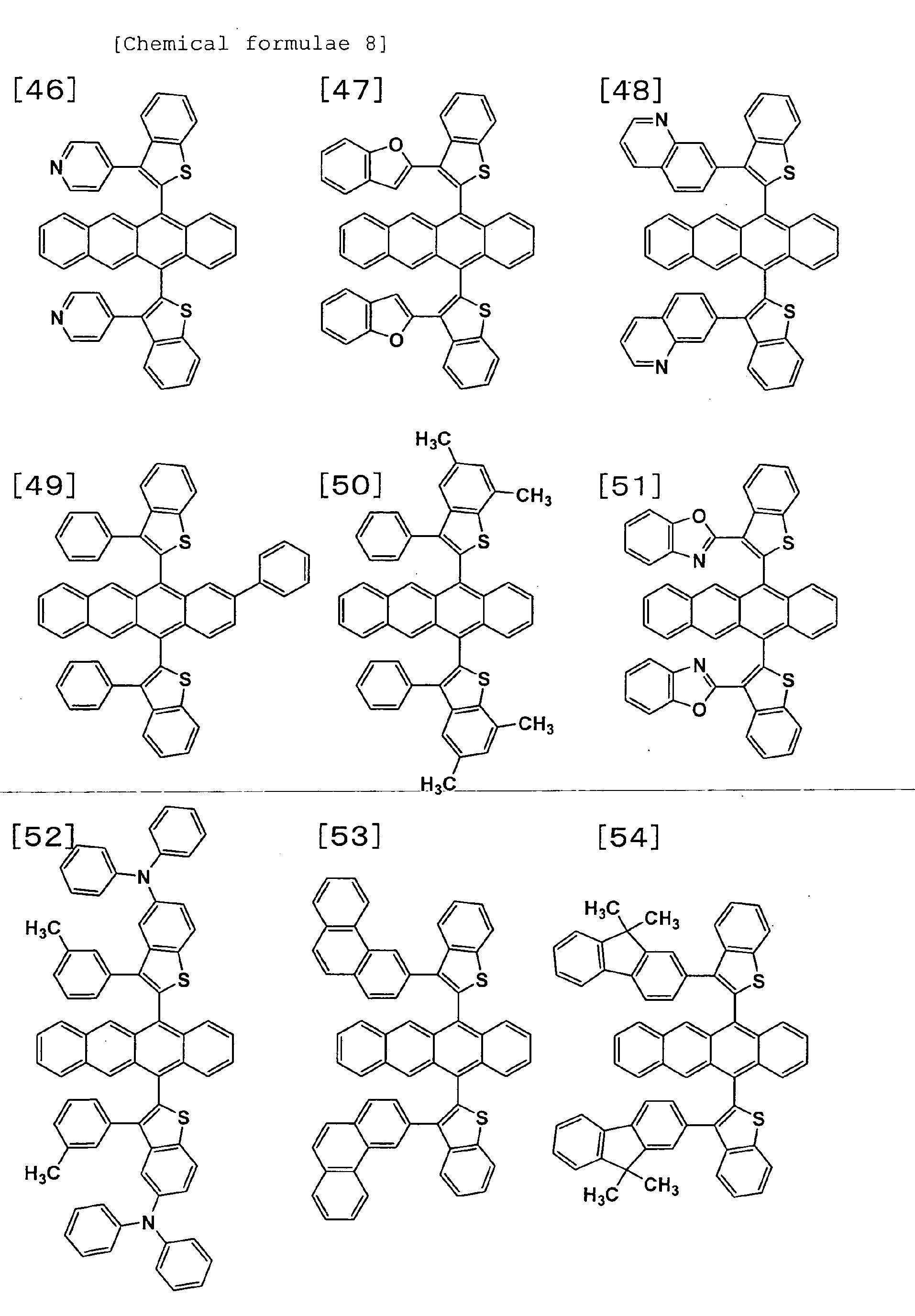

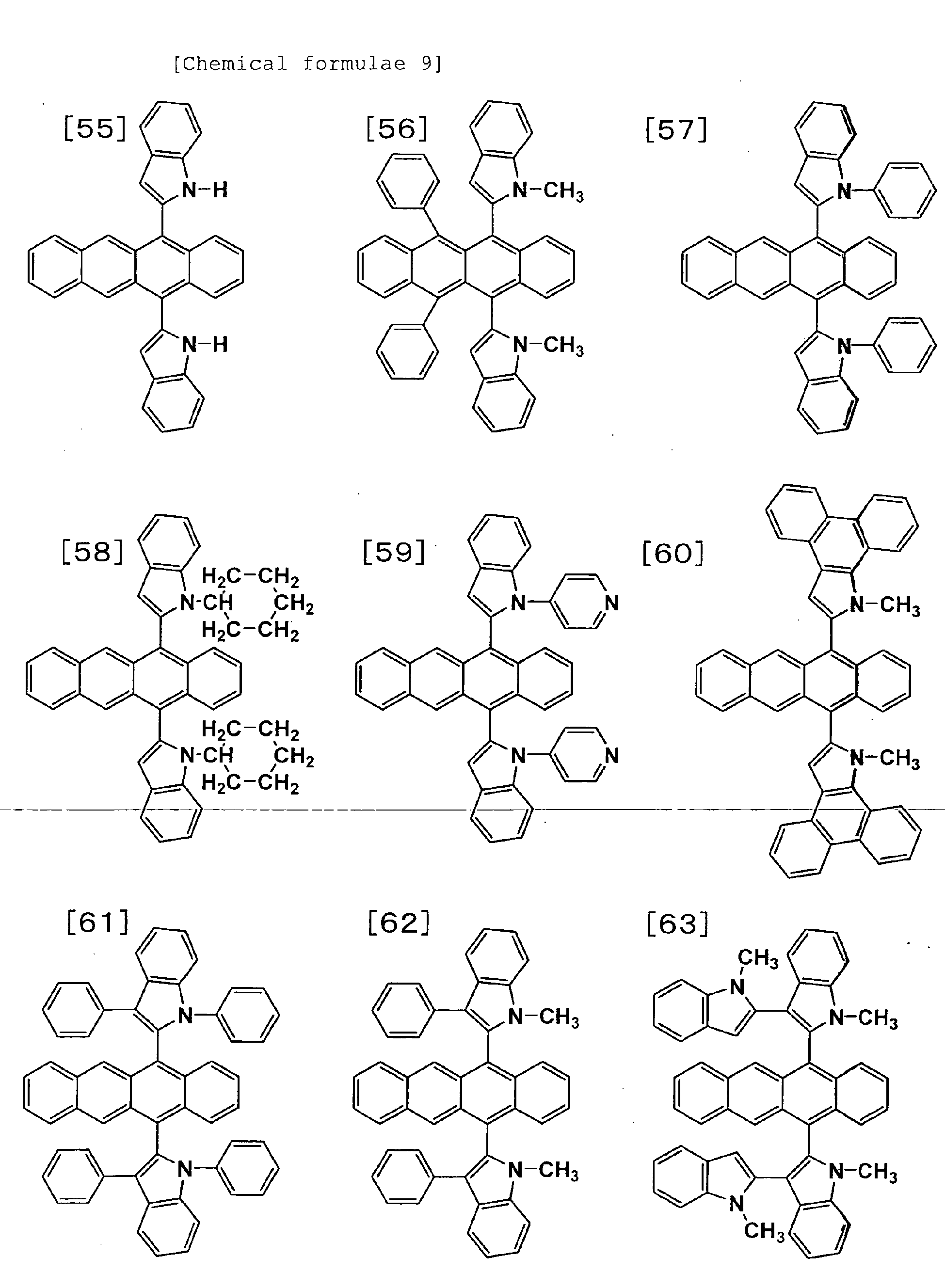

An embodiment of the light-emitting element of this invention is explained below in detail in reference to examples. The light-emitting element of this invention comprises at least an anode, a cathode and an organic layer composed of a light-emitting element material interposed between the anode and the cathode.

The anode used in this invention is not especially limited, if it is a material capable of efficiently injecting holes into an organic layer, but it is preferred to use a material with a relatively large work function. Examples of the material include electroconductive metal oxides such as tin oxide, indium oxide, indium zinc oxide and indium tin oxide (ITO), metals such as gold, silver and chromium, inorganic electroconductive substances such as copper iodide and copper sulfide, electroconductive polymers such as polythiophene, polypyrrole and polyaniline, etc. Any one of these electrode materials can be used alone, or multiple materials can also be used as a laminate or mixture.

With regard to the resistance of the anode, it is only required that an electric current sufficient for light emission of the light-emitting element can flow, and in view of the power consumption of the light-emitting element, low resistance is desired. Fro example, an ITO substance of 300 Ω/□ or less can function as an element electrode, but at present, a substrate of approx. 10 Ω/□ can also be supplied. Therefore, it is especially desired to use a low resistance substrate of 100 Ω/□ or less. The thickness of ITO can be arbitrarily selected in response to the resistance value, but usually the thickness is often selected in a range from 100 to 300 nm.

Further, in order tb keep the mechanical strength of the light-emitting element, it is preferred to form the light-emitting element on a substrate. As the substrate, a glass substrate of soda glass or non-alkali glass, etc. can be suitably used. The thickness of the glass substrate is only required to be a sufficient thickness for keeping the mechanical strength, and therefore 0.5 mm or more is sufficient. As the material of glass, non-alkali glass is preferred since it is desired that the amount of the ions dissolved from glass is smaller. Soda-lime glass coated with a barrier such as SiO₂ is also commercially available, and such glass can also be used. Further, if the anode can stably function, the substrate is not required to be glass, and for example, the anode can also be formed on a plastic substrate. The ITO film forming method is not especially limited, and an electron beam method, sputtering method, chemical reaction method or the like can be used.

The material used as the cathode of this invention is not especially limited, as far as the material allows electrons to be efficiently injected into the organic layer. Examples of the material generally include platinum, gold, silver, copper, iron, tin, zinc, aluminum, indium, chromium, lithium, sodium, potassium, cesium, calcium, magnesium, alloys thereof, etc. In order to enhance the electron injection efficiency, for enhancing the element properties, lithium, sodium, potassium, cesium, calcium, magnesium and alloys containing these low work function metals are effective. However, since these low work function metals are generally unstable in air, it is preferred to use a method of doping the organic layer with a slight amount of lithium or magnesium (1 nm or less as displayed on a vacuum-evaporated film thickness monitor) for obtaining a highly stable electrode. Further, an inorganic salt such as lithium fluoride can also be used. Furthermore, as a preferred example of protecting the electrode, a metal such as platinum, gold, silver, copper, iron, tin, aluminum or indium, an alloy consisting of these metals, an inorganic substance such as silica, titania or silicon nitride, or an organic polymer such as polyvinyl alcohol, polyvinyl chloride or hydrocarbon-based polymer can be laminated. The method for producing such an electrode is not especially limited, and resistance heating, electron beam, sputtering, ion plating, coating or the like can be used if conduction can be assured.

The light-emitting element of this invention is formed of a light-emitting element material comprising an organic layer containing the naphthacene compound represented by general formula (1). A light-emitting element material refers to a compound associated with light emission, being capable of emitting light or being capable of assisting light emission. Particularly it refers to a hole transport material, light-emitting material, electron transport material, etc.

The organic layer constituting the light-emitting element of this invention comprises at least a light-emitting layer having a light-emitting element material. The organic layer can consist of a light-emitting layer only or can also be a laminate consisting of (1) hole transport layer/light-emitting layer/electron transport layer, (2) light-emitting layer/electron transport layer, or (3) hole transport layer/light-emitting layer, etc. Further, each of the abovementioned layers can also be either a single layer or consists of multiple layers. In the case where a hole transport layer or an electron transport layer consists of multiple layers, the layer on the side kept in contact with an electrode may be called a hole injection layer or an electron injection layer respectively. In the following explanation, a hole injection material is contained in a hole transport material, and an electron injection material, in an electron transport material.

A hole transport layer is formed by a method of laminating or mixing one or more hole transport materials or a method of using a mixture consisting of a hole transport material and a polymeric binder. Further, a hole transport layer may also be formed by adding an inorganic salt such as iron(III) chloride to a hole transport material. A hole transport material is not especially limited, if it is a compound capable of forming a thin film necessary for producing a light-emitting element, allowing the injection of holes from an anode and capable of transporting the holes. Preferred examples of the hole transport material include 4,4'-bis(N-(3-methylphenyl)-N-phenylamino)biphenyl, 4,4'-bis(N-(1-naphthyl)-N-phenylamino)biphenyl, triphenylamine derivatives such as 4,4',4"-tris(3-methylphenyl)phenyl)amino)triphenylamine, biscarbazole derivatives such as bis(N-allylcarbazole) and bis (N-alkylcarbazole), pyrazoline derivatives, stilbene-based compounds, hydrazone-based compounds, benzofuran derivatives, thiophene derivatives, oxadiazole derivatives, phthalocyanine derivatives, heterocyclic compounds such as porphyrin derivatives, polymers such as polycarbonate having any of the aforementioned monomers at the side chains, styrene derivatives, polythiophene, polyaniline, polyfluorene, polyvinyl carbazole, polysilanes, etc. The hole transport material is not especially limited, if it is a compound capable of forming a thin layer necessary for producing a light-emitting element, allowing the injection of holes from an anode and further capable of transporting the holes.
In this invention, the light-emitting layer can be either a single layer or consists of multiple layers, and either type of the light-emitting layer can be formed of a light-emitting material containing a host material and, as required, a dopant material as main components. The light-emitting material can be either a mixture consisting of a host material and a dopant material or a host material alone. That is, in the light-emitting layer of the light-emitting element of this invention, a host material only or a dopant material only can emit light, or both a host material and a dopant material can emit light. The host material and the dopant material can respectively consist of a single material or a combination of multiple materials. The dopant material can be contained in the host material as a whole or partially in the host material. The dopant material can be either laminated or dispersed.
If the amount of the dopant material is too large, concentration quenching occurs. Therefore, it is preferred that the amount of the dopant material is 20 wt% or less based on the weight of the host material. More preferred is 10 wt% or less. As for the doping method, a co-deposition method with the host material can be used, but a mixture consisting of the doping material and the host material can also be used for simultaneous deposition.

The naphthacene compound represented by general formula (1) can also be used as a hole transport material or an electron transport material, but since it has high light emission performance, it can be suitably used as a light-emitting material. Further, since the light-emitting element material of this invention emits strong light in a bluish-green to red region (500 to 680 nm region), it can be suitably used as a bluish green to red light-emitting material. Furthermore, in the case where the light-emitting element material is used as a host-dopant light-emitting material, the naphthacene compound of this invention can also be used as a dopant material, but since it is excellent in thin film stability, it can be suitably used as a host material. If the naphthacene compound of this invention is used as a host material, green light emission or red light emission with high color purity can be obtained at high light' emission efficiency in response to the dopant material used in combination.

The host material used in this invention is not required to be limited only to one naphthacene compound represented by the general formula (1) of this invention, and multiple naphthacene compounds of this invention can also be used as a mixture or one or more other host materials can also be used as a mixture with a naphthacene compound of this invention. As other host materials that can be mixed, suitably used are light emitters including condensed ring derivatives such as anthracene and perylene, aromatic amine derivatives such as N,N'-dinaphthyl-N,N'-diphenyl-4,4'-diphenyl-1,1'-diamine, tris(8-quinolinate)aluminum (III) and other metal-chelated oxynoid compounds, bisstyryl derivatives such as distyryl benzene derivatives, tetraphenyl butadiene derivatives, indene derivatives, cumarine derivatives, oxadiazole derivatives, pyrrolopyridine derivatives, perinone derivatives, cyclopentadiene derivatives, carbazole derivatives, pyrrole-pyrrole derivatives, polymers such as polyphenylene vinylene derivatives, polyparaphenylene derivatives, polyfluorene derivatives, polyvinyl carbazole derivatives and polythiophene derivatives.

The dopant material contained in the light-emitting material is not especially limited, and examples of the dopant material include compounds having a condensed aromatic ring such as naphthacene, rubrene, perylene and Terylene, derivatives thereof, compounds having a heteroaryl ring such as thiophene, imidazopyridine, pyrazine, naphthyridine, quinoxaline, pyrrolopyridine, thioxanthene, pyridinothiadiazole and pyrazolopyridine, derivatives thereof, distyryl benzene derivatives, aminostyryl derivatives, aldazine derivatives, diketopyrrolo[3,4-c]pyrrole derivatives, cumarine derivatives such as 2,3,5,6-1H,4H-tetrahydro-9-(2'-benzothiazolyl)quinolidino[9,9a ,1-gh]cumarine, azole derivatives such as imidazole, thiazole, thiadiazole, carbazole, oxazole, oxadiazole and triazole, metal complexes thereof, aromatic amine derivatives, naphthalimide derivatives such as bis(diisopropylphenyl)perylene tetracarboxylic imide, perynone derivatives, rare earth complexes such as Eu complex with acetylacetone or benzoylacetone and phenanthroline, etc. as ligands, pyran derivatives such as 4-(dicyanomethylene)-2-methyl-6-(p-dimethylaminostyryl)-4H-pyr an, metal phthalocyanine derivatives such as magnesium phthalocyanine and aluminum chlorophthalocyanine, metal porphyrin derivatives such as zinc porphyrin, rhodamine compounds, deazaflavin derivatives, oxazine compounds, phenoxazine derivatives, phenoxazone derivatives, quinacridone derivatives, dicyanoethenyl allene derivatives, etc. Among them, a dopant material with a high fluorescence quantum yield as a solution can be preferably used since highly efficient light emission can be obtained. Further, in the case where it is combined with a naphthacene compound represented by general formula (1), an organic fluorescent material with a fluorescence peak wavelength of 500 nm to 680 nm is preferred, and further in view of energy transfer efficiency, a organic fluorescent material with a fluorescence peak wavelength of 500 nm to 650 nm is more preferred.

Among the abovementioned organic fluorescent materials, a compound with a pyrromethene skeleton represented by general formula (2) is especially preferred since light emission with high color purity can be obtained at high efficiency owing to a high fluorescence quantum yield and a narrow half value width of the fluorescence spectrum.

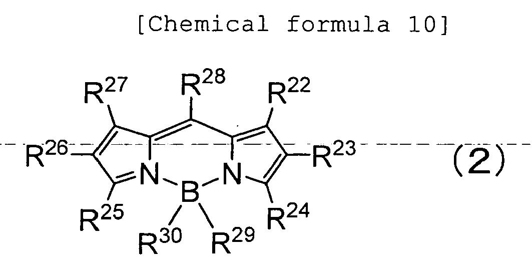

In the above formula, R²² to R³⁰ can be identical to or different from each other, and are respectively selected from hydrogen, alkyl group, cycloalkyl group, heterocyclic ring group, alkenyl group, cycloalkenyl group, alkynyl group, alkoxy group, alkylthio group, aryl ether group, aryl thioether group, aryl group, heteroaryl group, cyano group, amino group, silyl group, -P(=O)R³¹R³² or a ring structure formed with an adjacent substituent group. R³¹ and R³² are respectively selected from an aryl group and heteroaryl group. R²⁹ and R³⁰ can be identical to or different from each other and are respectively selected from a halogen, hydrogen, alkyl group, aryl group and heterocyclic ring group. Meanwhile, the substituent groups thereof are as explained before.

Since the abovementioned compound having a pyrromethene skeleton is excellent in compatibility with the naphthacene compound of this invention, the energy transfer between a host material and a dopant material occurs efficiently. For this reason, a light-emitting element with both high light emission efficiency and high durability can be obtained.

Among the pyrromethene metal complexes represented by general formula (2), those in which R²², R²⁴, R²⁵ and R²⁷ denote respectively an alkyl group or cycloalkyl group can be preferably used for particularly obtaining bluish green to green light emission. Among them, it is more preferred that those groups denote respectively an alkyl group with 1 to 8 carbon atoms or a cycloalkyl group with 3 to 8 carbon atoms. Further, those in which R²², R²⁴, R²⁵ and R²⁷ denote respectively an aryl group can be preferably used for particularly obtaining red light emission. Each of the aryl groups may also by substituted by an alkyl group with 1 to 6 carbon atoms, a cycloalkyl group with 3 to 8 carbon atoms, an alkoxy group with 1 to 6 carbon atoms, an aryl ether group with 6 to 14 carbon atoms, or an aryl group with 6 to 14 carbon atoms.

In the case where R²², R²⁴, R²⁵ and R²⁷ denote respectively a substituent group other than hydrogen, these groups can be identical to or different from each other, but in view of high color purity and high light emission efficiency, either R²² = R²⁴ and R²⁵ = R²⁷ or R²² = R²⁷ and R²⁴ = R²⁵ are preferred. Meanwhile, R²² = R²⁴ mean that R²² denotes the same group as R²⁴.

Examples of the pyrromethene metal complex represented by the general formula (2) as described above include the following.

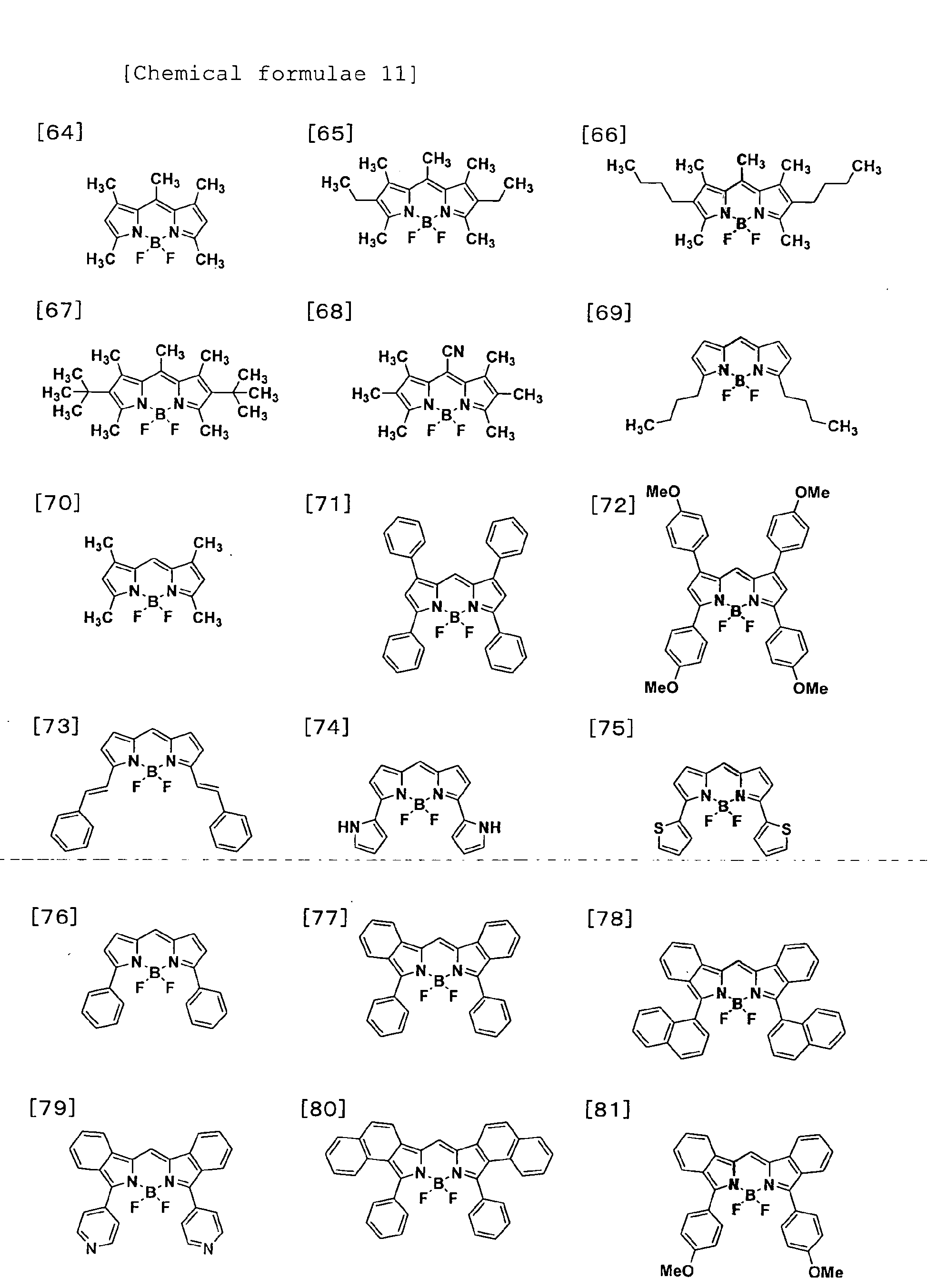

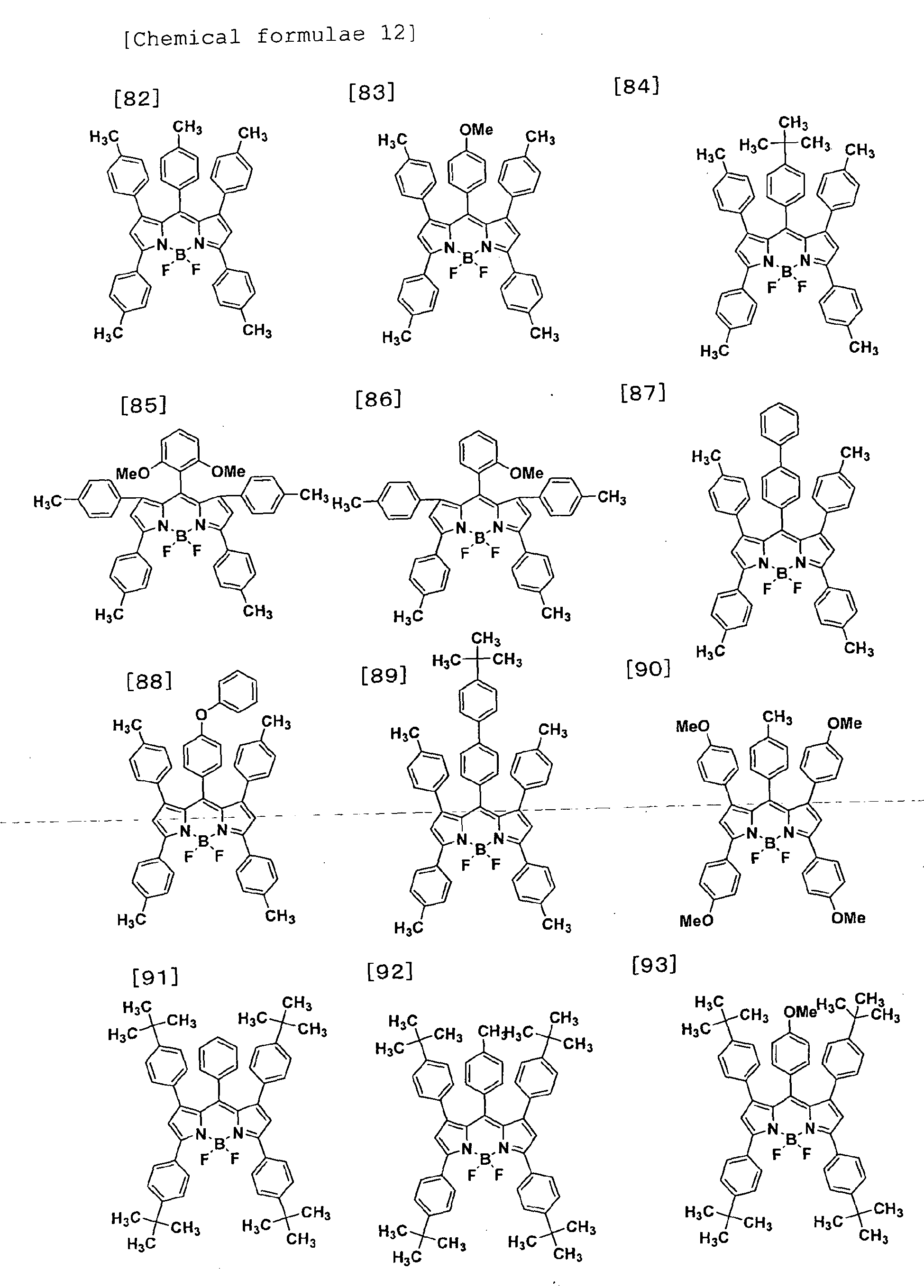

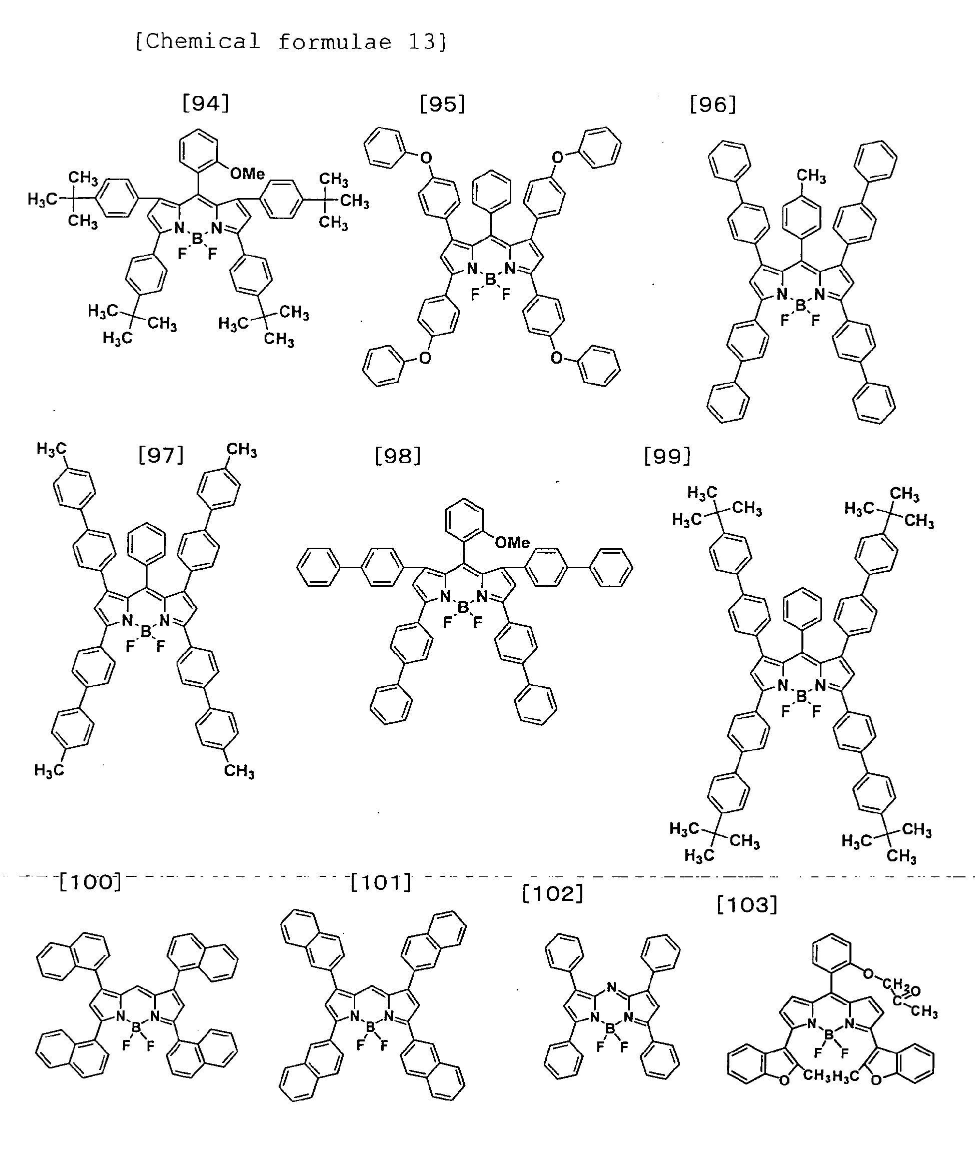

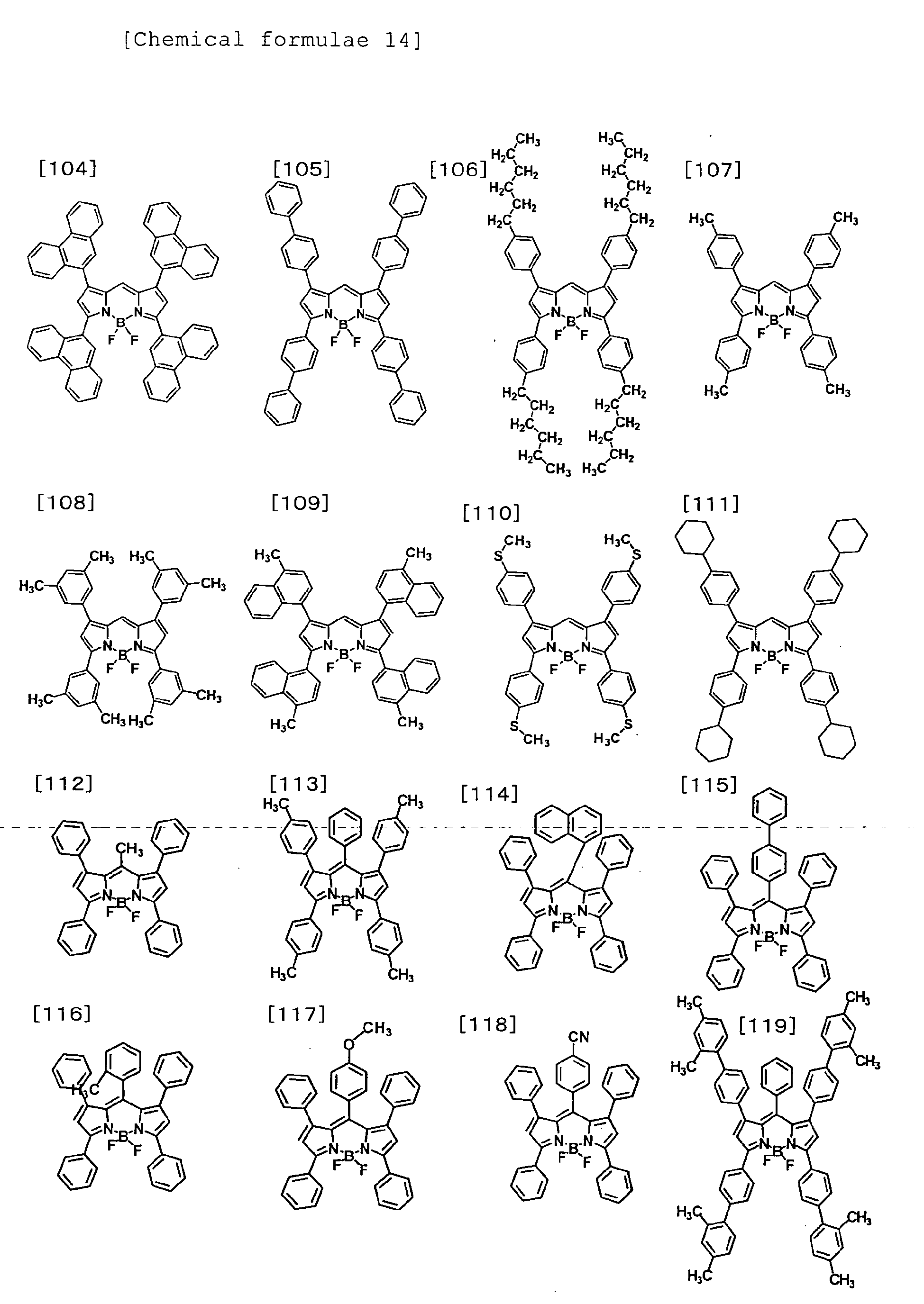

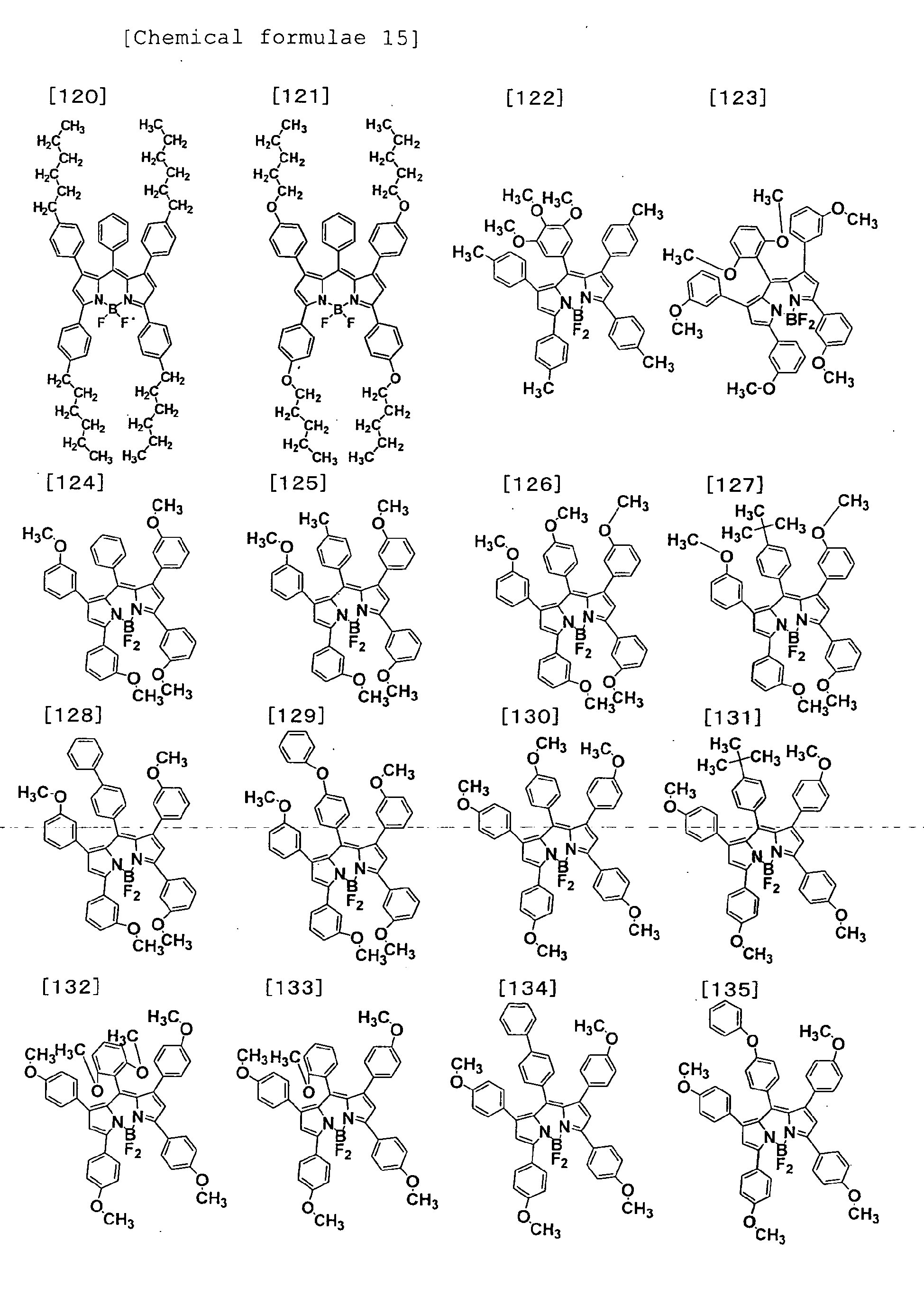

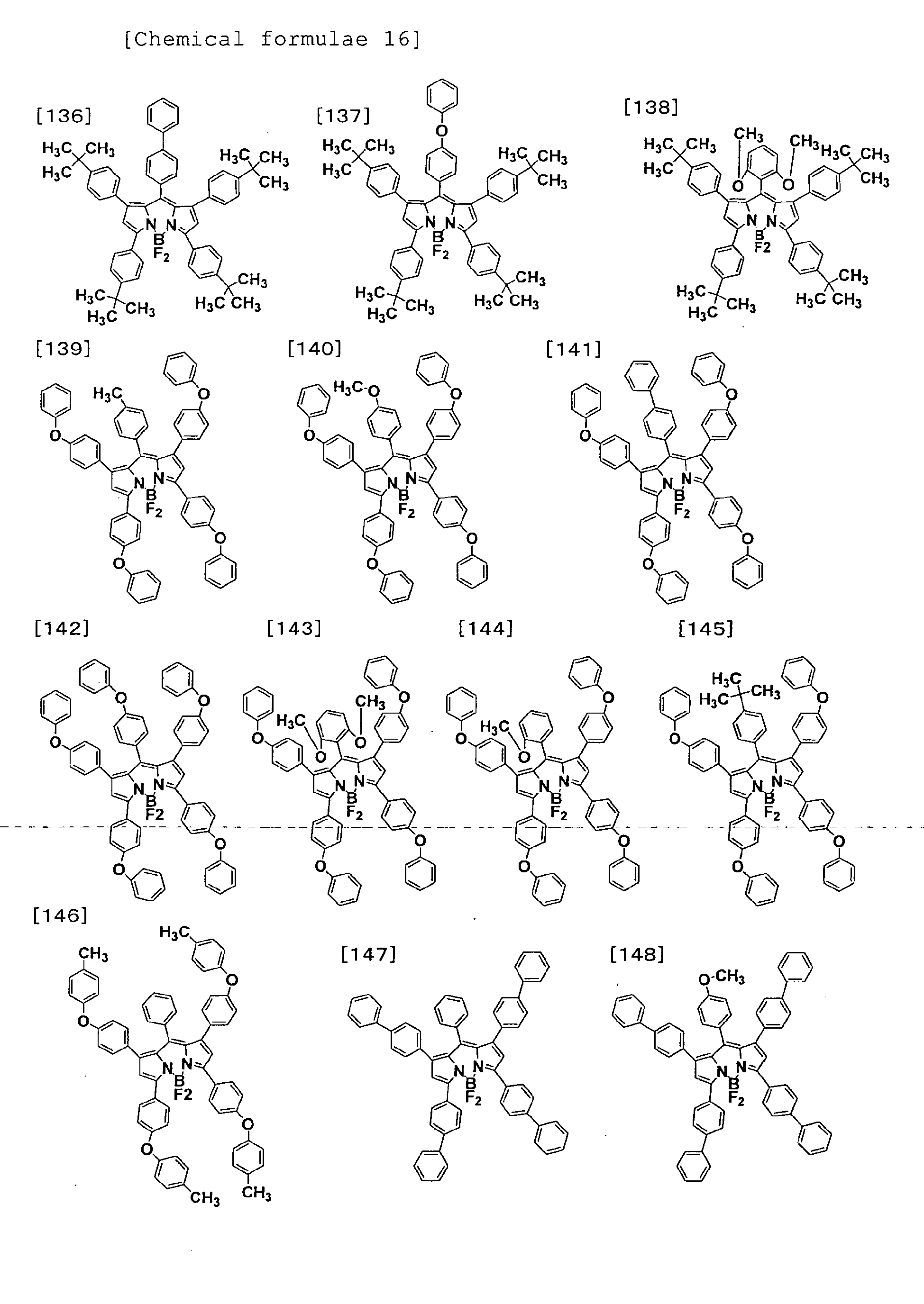

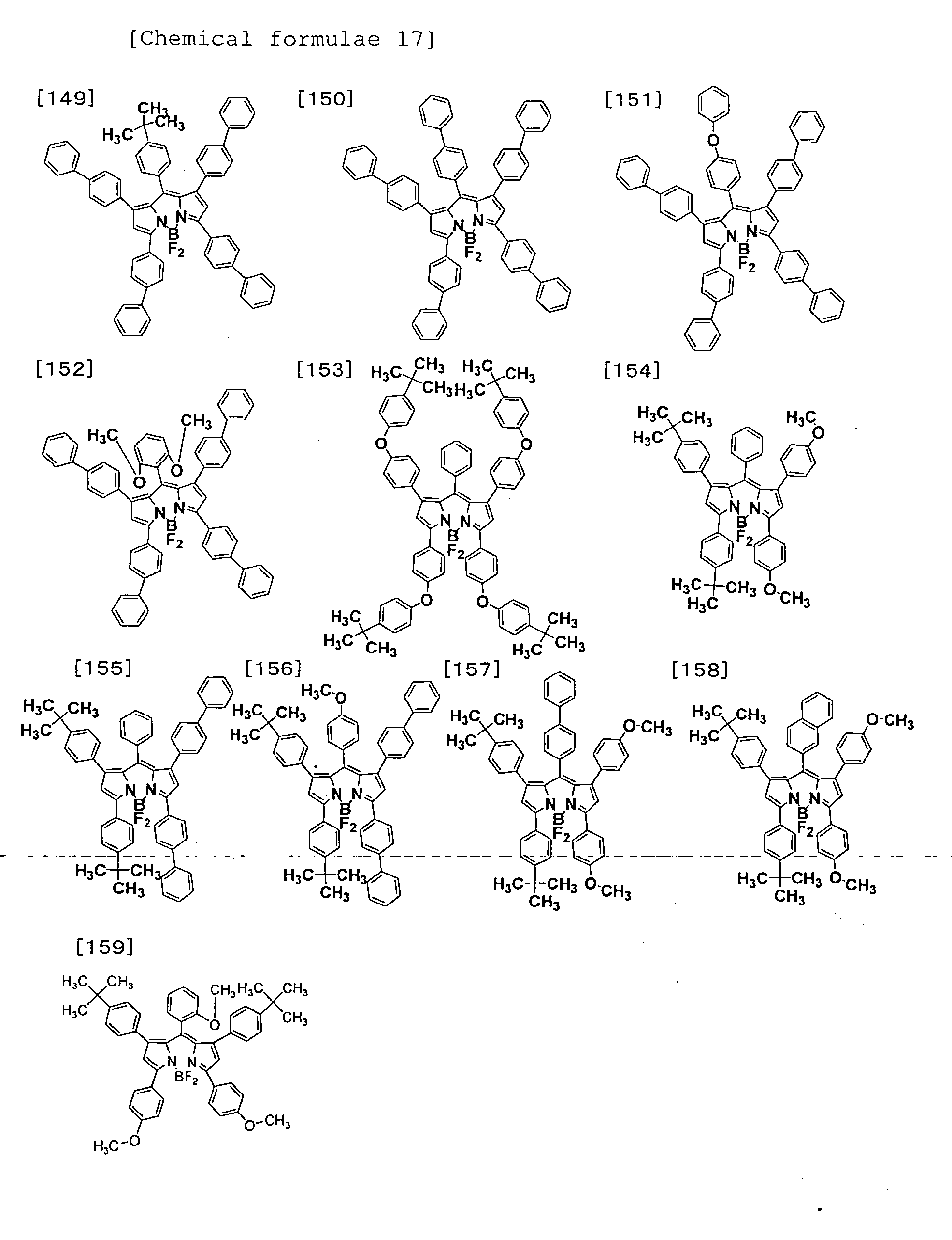

The dopant material contained in the light-emitting material is not limited to only one compound having the aforementioned pyrromethene skeleton, and multiple pyrromethene compounds can also be used as a mixture, or one or more of the abovementioned dopant materials can also be used as a mixture with a pyrromethene compound.

In this invention, an electron transport layer refers to a layer for having electrons injected from the cathode and for transporting the electrons. The electron transport layer is desired to be high in electron injection efficiency and to efficiently transport the injected electrons. For this reason, the electron transport layer is desired to be formed of a material large in electrone affinity, large in electron mobility, excellent in stability and unlikely to general impurities destined to be traps during production and during use. However, in the case where the transport balance between holes and electrons is taken into consideration, if the electron transport layer mainly plays the role of efficiently inhibiting the flow of holes from the anode to the cathode without re-coupling, the effect of enhancing light emission efficiency of the electron transport layer formed of a material not so high in electron transport capability is equivalent to that of the electron transport layer formed of a material high in electron transport capability. Therefore, a hole inhibition layer capable of efficiently inhibiting the migration of holes is considered to be a synonym of the electron transport layer of this invention.

As examples of the electron transport material used in the electron transport layer, enumerated are condensed polycyclic aromatic derivatives such as naphthalene and anthracene, styryl-based aromatic ring derivatives typified by 4,4'-bis(diphenylethenyl)biphenyl, quinone derivatives such as anthraquinone and diphenoquinone, phosphorus oxide derivatives, quinolinol complexes such as tris(8-quinolinolate)aluminum (III), benzoquinolinol complexes, hydroxyazole complexes, azomethine complexes and various metal complexes such as tropolone metal complex and flavonol metal complex. For such reasons that the drive voltage can be decreased and that highly efficient light emission can be obtained, it is preferred to use a compound composed of elements selected from carbon, hydrogen, nitrogen, oxygen, silicon and phosphorus and having a heteroaryl ring structure containing electron-acceptable nitrogen.

The electron-acceptable nitrogen in this invention refers to a nitrogen atom forming a multiple bond with an adjacent atom. Since a nitrogen atom has high electron negativity, the multiple bond has an electron-acceptable nature. Therefore, a heteroaryl ring containing an electron-acceptable nitrogen has high electron affinity and excellent electron transport capability, and if it is used in an electron transport layer, the drive voltage of the light-emitting element can be decreased. As examples of the heteroaryl ring containing an electron-acceptable nitrogen, enumerated are a pyridine ring, pyrazine ring, pyrimidine ring, quinoline ring, quinoxaline ring, naphthyridine ring, pyrimidopyrimidine ring, benzoquinoline ring, phenanthroline ring, imidazole ring, oxazole ring, oxadiazole ring, triazole ring, thiazole ring, thiadiazole ring, benzooxazole ring, benzothiazole ring, benzimidazole ring, phenanthroimidazole ring, etc.

As examples of the compound having any of these heteroaryl ring structures, enumerated are benzimidazole derivatives, benzooxazole derivatives, benzthiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives such as bipyridine and terpyridine, quinoxaline derivatives, naphthyridine derivatives, etc. Among them, in view of electron transport capability, preferably used are imidazole derivatives such as tris (N-phenylbenzimidazole-2-yl)benzene, oxadiazole derivatives such as 1,3-bis[(4-tert-butylphenyl)1,3,4-oxadiazolyl]phenylene, triazole derivatives such as N-naphthyl-2,5-diphenyl-1,3,4-triazole, phenanthroline derivatives such as bathocuproin and 1,3-bis-(1,10-phenanthrolin-9-yl)benzene, benzoquinoline derivatives such as 2,2'-bis(benzo[h]quinoline-2-yl)-9,9'-spirobifluorene, bipyridine derivatives such as 2,5-bis(6'-(2',2"-bipyridyl)-1,1-dimethyl-3,4-diphenylsilole, terpyridine derivatives such as 1,3-bis(4'-(2,2':6',2"-terpyridinyl))benzene and naphthyridine derivatives such as bis(1-naphthyl)-4-(1,8-naphthyridine-2-yl)phenylphosphine oxide.

Any one of the abovementioned electron transport materials can be used alone, or two or more of the abovementioned electron transport materials can also be used as a mixture. Otherwise, one or more other electron transport materials can also be used as a mixture with the abovementioned electron transport material(s). Further, the electron transport material(s) can also be used as a mixture with a metal such as an alkali metal or alkaline earth metal. The ionization potential of the electron transport layer is not especially limited. A preferred range of the ionization potential is 5.8 eV to 8.0 eV, and a more preferred range is 6.0 eV to 7.5 eV.

The methods for forming the respective layers constituting the light-emitting element are not especially limited and include resistance heating vapor deposition, electron beam vapor deposition, sputtering, molecular lamination method, coating method, ink jet method, printing method, laser-induced heat transfer method, etc. Usually in view of element properties, resistance heating vapor deposition or electron beam vapor deposition is preferred.

The thickness of each layer cannot be limited, since it also depends on the resistance value of the light-emitting material, and is selected in a range from 1 to 1,000 nm. It is preferred that the thickness of the light-emitting layer, electron transport layer and hole transport layer are respectively 1 nm to 200 nm. A more preferred range is 5 nm to 100 nm.

The light-emitting element of this invention can convert electric energy into light. In this case, the electric energy mainly refers to a DC current, but a pulse current or AC current can also be used. The current value and the voltage value are not especially limited, but considering the power consumption and life of the element, efforts should be made to obtain the largest luminance with the lowest possible energy.

The light-emitting element of this invention can be suitably used, for example, in a display for displaying with a matrix and/or segment system.

The matrix system in this invention refers to a system in which the pixels used for displaying are arranged two-dimensionally like a lattice or mosaic, and a set of pixels is used for displaying a character or image. The form and size of a pixel are decided for each application. For example, in order to display an image and characters on a personal computer, monitor or TV set, a square of 300 µm or less per side is as the form of each pixel, and in the case of a large display like a display panel, each pixel is in the order of mm per side. In the case of a monochromatic display, pixels of the same color can be arranged, but in the case of a color display, red, green and blue pixels are arranged for displaying. In the latter case, typically delta type and stripe type are available. Further, as the matrix drive method, either line-sequential drive method or active matrix drive method can be used. The line-sequential drive method has an advantage that the structure is simple, but considering the action characteristics, the active matrix drive method may be more excellent as the case may be. Therefore, it is necessary to selectively use the drive method suitable for each application.

In the segment system of this invention, a pattern is formed to display predetermined information, and determined regions emit light. Examples of the segment system include the time displayed on a digital timepiece, the temperature displayed on a thermometer, an operation state displayed on an audio apparatus or electromagnetic cooker, a panel display of an automobile, etc. Further, the aforementioned matrix display and segment display can also coexist on the same panel.

The light-emitting element of this invention can be preferably used also in the backlights of various apparatuses, etc. A backlight is used for the purpose of enhancing the visibility of a display device which per se does not emit light, and is used in a liquid crystal display device, timepiece, audio apparatus, automobile panel, display board, signboard, etc. Especially considering that the conventional backlight uses a fluorescent lamp or light guide plate difficult to be thinned, the backlight using the light-emitting elements of this invention can be thin in thickness and light in weight, and therefore can be applied to a liquid crystal display device, above all, a personal computer which is required to be as thin as possible.

### EXAMPLES

This invention is explained below in reference to examples, but is not limited thereto or thereby. Meanwhile, the compound numbers in the following examples refer to the compound numbers stated before.

### Synthesis Example 1

### Synthesis of compound [7]

Nine point five grams of phenacyl bromide, 6.8 g of phenol and 13.3 g of potassium carbonate were dissolved into 192 mL of acetone, and the solution was stirred and heated under reflux in a nitrogen stream for 3 hours. It was cooled to room temperature, and subsequently 200 mL of water was added. The mixed solution was subjected to extraction with 150 mL of dichloromethane. The organic layer was washed with 100 mL of water twice and dried with magnesium sulfate, the solvent being distilled off by a rotary evaporator. The obtained reaction product composition was purified by silica gel chromatography and dried in vacuum to obtain 9.7 g of a yellow liquid (yield 86%). The ¹H-NMR analysis result of the obtained liquid was as shown below, and it was confirmed that the obtained yellow liquid was 2-phenoxy-1-phenylethanone.
¹H-NMR (CDCl₃ (d=ppm)) : 5.27(s, 2H), 6.87-7.01(m, 2H), 7.19-7.33(m, 2H), 7.47-7.65(m, 4H), 7.96-8.03(m, 2H).

Nine point seven grams of 2-phenoxy-1-phenylethanone was dissolved into 200 mL of dichloromethane, and 22.0 g of methanesulfonic acid was added dropwise in a nitrogen stream. Subsequently the mixed solution was stirred and heated under reflux for 1 hour. The solution was cooled to room temperature, and subsequently 200 mL of water was added, the mixed solution being subjected to extraction with 250 mL of dichloromethane. The organic layer was washed with 100 mL of water twice and dried with magnesium sulfate, the solvent being distilled off by a rotary evaporator. The obtained reaction product composition was purified by silica gel column chromatography and dried in vacuum, to obtain 6.9 g of a light yellow liquid (yield 78%). The ¹H-NMR analysis result of the obtained liquid was as shown below, and it was confirmed that the obtained light yellow liquid was 3-phenylbenzofuran. ¹H-NMR (CDCl₃ (d=ppm)): 7.22-7.79(m, 8H) , 7. 82(s, 1H) , 7.84-7.88(m, 1H).

Six point zero grams of 3-phenylbenzofuran was dissolved into 124 mL of dehydrated ether, and 23.2 mL of n-butyllithium (1.6M hexane solution) was added dropwise at 0°C in a nitrogen stream, the mixed solution being stirred at 0°C for 1 hour. Subsequently 20 mL of dehydrated ether solution containing 2.9 g of 5, 12-naphthacene quinone was added dropwise, and the mixed solution was stirred at room temperature for 1 hour, and then stirred and heated under reflux for further 1 hour. The solution was cooled to room temperature, and 50 mL of 0.1M hydrochloric acid aqueous solution was added. The mixed solution was stirred at room temperature for 1 hour and subjected to extraction with 250 mL of toluene. The organic layer was washed with 100 mL of water twice and dried with magnesium sulfate, the solvent being distilled off by a rotary evaporator. The obtained crude reaction product was dried in vacuum, to obtain 6.8 g of a reddish orange liquid (yield 94%).

Six point eight grams of the abovementioned crude reaction product was dissolved into 210 mL of tetrahydrofuran, and 50 mL of 35% hydrochloric acid solution containing 4.7 g of tin(II) chloride dihydrate was added dropwise at room temperature, the mixed solution being stirred and heated under reflux for 9 hours. The solution was cooled to room temperature, and subsequently the precipitated powder was collected by filtration and washed with 50 mL of methanol. The obtained powder was stirred and heated under reflux with 30 mL of acetone, and the mixture was cooled to room temperature, the precipitated powder being collected by filtration. Subsequently the obtained powder was stirred with 25 mL of cyclopentyl methyl ether at 60°C for 1 hour and the mixture was cooled to room temperature, the precipitated powder being collected by filtration. The obtained powder was dried in vacuum, to obtain 3.8 g of an orange powder (yield 59%). The ¹H-NMR analysis result of the obtained powder was as shown below, and it was confirmed that the obtained orange powder was compound [7].
¹H-NMR (CDCl₃ (d=ppm)): 7.06-8.29(m, 26H), 8.50(s, 2H).

Meanwhile, the compound [7] was sublimed and purified at a pressure of 1 x 10⁻³ Pa and at approx. 230°C using an oil diffusion pump, to be used as a light-emitting element material. HPLC purity (area % at a measurement wavelength of 254 nm) was 99.0% before the sublimation purification and 99.4% after the sublimation purification.

### Synthesis Example 2

### Synthesis of compound [1]

Five point zero grams of benzofuran was dissolved into 169 mL of dehydrated ether, and 31.7 mL of n-butyllithium (1.6M hexane solution) was added dropwise at 0°C in a nitrogen stream, the mixed solution being stirred at 0°C for 1 hour. Then, 30 mL of dehydrated ether solution containing 3.9 g of 5,12-naphthacene quinone was added dropwise, and the mixed solution was stirred at room temperature for 1 hour, and subsequently stirred and heated under reflux for further 1 hour. The solution was cooled to room temperature, and subsequently stirred and heated under reflux for further 1 hour. The solution was cooled to room temperature, and subsequently 70 mL of 0.1M hydrochloric acid aqueous solution was added, the mixed solution being stirred at room temperature for 1 hour and subjected to extraction with 300 mL of toluene. The organic layer was washed with 100 mL of water twice and dried with magnesium sulfate, the solvent being distilled off by a rotary evaporator. The obtained crude reaction product was dried in vacuum, to obtain 6.7 g of a reddish orange liquid (yield 90%).

Six point seven grams of the abovementioned crude reaction product was dissolved into 210 mL of tetrahydrofuran, and 60 mL of 35% hydrochloric acid solution containing 6.1 g of tin(II) chloride dihydrate was added dropwise at room temperature, the mixed solution being stirred and heated under reflux for 9 hours. The solution was cooled to room temperature, and the precipitated powder was collected by filtration and washed with 50 mL of methanol. The obtained powder was stirred and heated under reflux with 30 mL of acetone for 1 hour and the mixture was cooled to room temperature, the precipitated powder being collected by filtration. Subsequently the obtained powder was stirred with 30 ml of cyclopentyl methyl ether at 60°C for 1 hour, and the mixture was cooled to room temperature, the precipitated powder being collected by filtration. The obtained powder was dried in vacuum, to obtain 3.4 g of an orange powder (yield 55%). The ¹H-NMR analysis result of the obtained powder was as shown below, and it was confirmed that the obtained orange powder was confirmed to be compound [1].
¹H-NMR (CDCl₃ (d=ppm)): 6.72(s, 2H), 7.10-7.91(m, 16H), 8.31(s, 2H).

Meanwhile, the compound [1] was sublimed and purified at a pressure of 1 x 10⁻³ Pa and at approx. 220°C using an oil diffusion pump, to be used as a light-emitting element material. HPLC purity (area % at a measurement wavelength of 254 nm) was 99.2% before the sublimation purification and 99.5% after the sublimation purification.

### Synthesis Example 3

### Synthesis of compound [16]

Nine point five grams of phenacyl bromide, 8.1 g of 4-hydroxybiphenyl and 13.3 g of potassium carbonate were dissolved into 192 mL of acetone, and the solution was stirred and heated under reflux in a nitrogen stream for 3 hours. The solution was cooled to room temperature and subsequently 200 mL of water was added, the mixed solution being subjected to extraction with 150 mL of dichloromethane. The organic layer was washed with 100 mL of water twice and dried with magnesium sulfate, the solvent being distilled off by a rotary evaporator. The obtained reaction product composition was purified by silica gel column chromatography and dried in vacuum, to obtain 12. 4 g of a yellow liquid (yield 90%). The ¹H-NMR analysis result of the obtained liquid was as shown-below, and it was confirmed that the obtained yellow liquid was 2-(biphenyl-4-yl-oxy)-1-phenylethanone.
¹H-NMR (CDCl₃ (d=ppm)): 5.20(s, 2H), 6.83-7.02(m, 2H), 7.22-7.48(m, 10H), 7.86-8.01(m, 2H).

Twelve point four grams of 2-(biphenyl-4-yl-oxy)-1-phenylethanone was dissolved into 250 mL of dichloromethane, and 20.7 g of methanesulfonic acid was added dropwise in a nitrogen stream, the mixed solution being stirred and heated under reflux for 1 hour. The solution was cooled to room temperature, and subsequently 250 mL of water was added, the mixed solution being subjected to extraction with 300 mL of dichloromethane. The organic layer was washed with 120 mL of water twice and dried with magnesium sulfate, the solvent being distilled off by a rotary evaporator. The obtained reaction product composition was purified by silica gel column chromatography and dried in vacuum, to obtain 9.3 g of a light yellow liquid (yield 80%). The ¹H-NMR analysis result of the obtained liquid was as shown below, and it was confirmed that the obtained light yellow liquid was 3,5-diphenylbenzofuran.
¹H-NMR (CDCl₃ (d=ppm)): 7.22-7.48(m, 12H) , 7. 50(s, 1H) , 7.71(s, 1H) .

Nine point three grams of 3,5-diphenylbenzofuran was dissolved into 138 mL of dehydrated ether, and 25.8 mL of n-butyllithium (1.6M hexane solution) was added dropwise at 0°C in a nitrogen stream, the mixed solution being stirred at 0°C for 1 hour. Then, 25 mL of dehydrated ether solution containing 3.2 g of 5,12-naphthacene quinone was added dropwise, and the mixed solution was stirred at room temperature for 1 hour and subsequently stirred and heated under reflux for further 1 hour. The solution was cooled to room temperature, and 55 mL of 0. 1M hydrochloric acid aqueous solution was added, the mixed solution being stirred at room temperature for 1 hour and subjected to extraction with 250 mL of toluene. The organic layer was washed with 100 mL of water twice and dried with magnesium sulfate, the solvent being distilled off by a rotary evaporator. The obtained crude reaction product was dried in vacuum, to obtain 9.5 g of a reddish orange liquid (yield 96%).

Nine point five grams of the abovementioned crude reaction product was dissolved into 238 mL of tetrahydrofuran, and 60 mL of 35% hydrochloric acid solution containing 5.4 g of tin(II) chloride dihydrate was added dropwise at room temperature, the mixed solution being stirred and heated under reflux for 9 minutes. The solution was cooled to room temperature, and subsequently the precipitated powder was collected by filtration and washed with 70 mL of methanol. The obtained powder was stirred and heated under reflux with 40 mL of acetone for 1 hour, and the mixture was cooled to room temperature, the precipitated powder being collected by filtration. Subsequently the obtained powder was stirred with 35 mL of cyclopentyl methyl ether at 60°C for 1 hour, and the mixture was cooled to room temperature, the precipitated powder being collected by filtration. The obtained powder was dried in vacuum, to obtain 5.4 g of an orange powder (yield 60%) . The ¹H-NMR analysis result of the obtained powder was as shown below, and it was confirmed that the obtained orange powder was compound [16]. ¹H-NMR (CDCl₃ (d=ppm)): 7.22-7.91(m, 34H), 8.31(s, 2H).

Meanwhile, the compound [16] was sublimed and purified at a pressure of 1 x 10⁻³ and at approx. 280°C using an oil diffusion pump, to be used as a light-emitting element material. HPLC purity (area % at a measurement wavelength of 254 nm) was 99.4% before the sublimation purification and 99.6% after the sublimation purification.

### Synthesis Example 4

### Synthesis of compound [18]

Nine point five grams of phenacyl bromide, 6.9 g of 2-hydroxynaphthalene and 13.3 g of potassium carbonate were dissolved into 192 mL of acetone, and the mixed solution was stirred and heated under reflux in a nitrogen stream for 3 hours. The solution was cooled to room temperature, and 200 mL of water was added, the mixed solution being subjected to extraction with 150 mL of dichloromethane. The organic layer was washed with 100 mL of water twice and dried with magnesium sulfate, the solvent being distilled off by a rotary evaporator. The obtained reaction product composition was purified by silica gel column chromatography and dried in vacuum, to-obtain 10.6 g of a yellow liquid (yield 85%). The ¹H-NMR analysis result of the obtained liquid was as shown below, and it was confirmed that the obtained yellow liquid was 2-(naphthalene-2-yl-oxy)-1-phenylethanone.
¹H-NMR (CDCl₃ (d=ppm)): 5.30(s,2H), 6.97-7.86(m, 12H).

Ten point six grams of 2-(naphthalene-2-yl-oxy)-1-phenylethanone was dissolved into 200 mL of dichloromethane, and 19.4 g of methanesulfonic acid was added dropwise in a nitrogen stream, the mixed solution being stirred and heated under reflux for 1 hour. The solution was cooled to room temperature, and then 200 mL of water was added, the mixed solution being subjected to extraction with 250 mL of dichloromethane. The organic layer was washed with 100 mL of water twice and dried with magnesium sulfate, the solvent being distilled off by a rotary evaporator. The obtained reaction product composition was purified by silica gel column chromatography and dried in vacuum, to obtain 4.7 g of a light yellow liquid (yield 48%). The ¹H-NMR analysis result of the obtained liquid was as shown below, and it was confirmed that the obtained light yellow liquid was 1-phenyl-naphtho[2,1-b]furan.
¹H-NMR (CDCl₃ (d=ppm)): 7.22-7.48(m, 9H), 7.51(s, 1H) , 7.67(d, 2H).

Four point seven grams of 1-phenyl-naphtho[2,1-b]furan was dissolved into 77 mL of dehydrated ether, and 14.4 mL of n-butyllithium (1.6M hexane solution) was added dropwise at 0°C in a nitrogen steam, the mixed solution being stirred at 0°C for 1 hour. Subsequently 10 mL of dehydrated ether solution containing 1.8 g of 5,12-naphthacene quinone was added dropwise, and the mixed solution was stirred at room temperature for 1 hour and then stirred and heated under reflux for further 1 hour. The solution was cooled to room temperature, and subsequently 30 mL of 0.1M hydrochloric acid aqueous solution was added, the mixed solution being stirred at room temperature for 1 hour and subjected to extraction with 200 mL of toluene. The organic layer was washed with 100 mL of water twice and dried with magnesium sulfate, the solvent being distilled off by a rotary evaporator. The obtained crude reaction product was dried in vacuum, to obtain 4.6 g of a reddish orange liquid (yield 88%).

Four point six grams of the abovementioned crude reaction product was dissolved into 123 mL of tetrahydrofuran, and 25 mL of 35% hydrochloric acid solution containing 2.8 g of tin(II) chloride dihydrate was added dropwise at room temperature, the mixed solution being stirred and heated under reflux for 9 hours. The solution was cooled to room temperature, and subsequently the precipitated powder was collected by filtration and washed with 40 mL of methanol. The obtained powder was stirred and heated under reflux with 25 mL of acetone for 1 hour and the mixture was cooled to room temperature, the precipitated powder being collected by filtration. Subsequently the obtained powder was stirred with 20 mL of cyclopentyl methyl ether at 60°C for 1 hour, and the mixture was cooled to room temperature, the precipitated powder being collected by filtration. The obtained powder was dried in vacuum, to obtain 2.5 g of an orange powder (yield 57%). The ¹H-NMR analysis result of the obtained powder was as shown below, and it was confirmed that the obtained orange powder was compound [18]. ¹H-NMR (CDCl₃ (d=ppm)): 7.19-8.03(m, 30H), 8.36(s, 2H).

Meanwhile, the compound [18] was sublimed and purified at a pressure of 1 x 10⁻³ Pa and at approx. 270°C using an oil diffusion pump, to be used as a light-emitting element material. HPLC purity (area % at a measurement wavelength of 254 nm) was 99.0% before the sublimation purification and 99.2% after the sublimation purification.

### Synthesis Example 5

### Synthesis of compound [6]

Five point five grams of an orange powder (yield 46%) was obtained by synthesizing according to the same method as in Synthesis Example 2, except that 6,11-diphenyl-5,12-naphthacene quinone was used as an alternative to 5,12-naphthacene quinone. The ¹H-NMR analysis result of the obtained powder was as shown below, and it was confirmed that the obtained orange powder was compound [6].
¹H-NMR (CDCl₃ (d=ppm)): 6.72(s, 2H), 7.11-7-91(m, 26H).

Meanwhile, the compound[6] was sublimed and purified at a pressure of 1 x 10⁻³ and at approx. 290°C using an oil diffusion pump, to be used as a light-emitting element material. HPLC purity (area % at a measurement wavelength of 254 nm) was 99.2% before the sublimation purification and 99.5% after the sublimation purification.

### Synthesis Example 6

### Synthesis of compound [21]

Four point one grams of an orange powder (yield 56%) was obtained by synthesizing according to the same method as in Synthesis Example 1, except that 3-(2-benzofuranyl)benzofuran was used as an alternative to 3-phenylbenzofuran. The ¹H-NMR analysis result of the obtained powder was as shown below, and it was confirmed that the obtained orange powder was compound [21]. ¹H-NMR (CDCl₃ (d=ppm)): 6.85-8.25(m, 26H), 8.48(s, 2H).

Meanwhile, the compound [21] was sublimed and purified at a pressure of 1 x 10⁻³ and at approx. 260°C using an oil diffusion pump, to be used as a light-emitting element material. HPLC purity (area % at a measurement wavelength of 254 nm) was 99.0% before the sublimation purification and 99.2% after the sublimation purification.

### Synthesis Example 7

### Synthesis of compound [22]

Three point seven grams of an orange powder (yield 48%) was obtained by synthesizing according to the same method as in Synthesis Example 1, except that 3-(2-naphthyl)-5-methylbenzofuran was used as an alternative to 3-phenylbenzofuran. The ¹H-NMR analysis result of the obtained powder was as shown below, and it was confirmed that the obtained orange powder was compound [22].
¹H-NMR (CDCl₃ (d=ppm)) : 2.35(s, 6H) , 6.98-8.25(m, 28H) , 8.48(s, 2H).

Meanwhile, the compound[22] was sublimed and purified at a pressure of 1 x 10⁻³ and at approx. 290°C using an oil diffusion pump, to be used as a light-emitting element material. HPLC purity (area % at a measurement wavelength of 254 nm) was 99.1% before the sublimation purification and 99.3% after the sublimation purification.

### Synthesis Example 8

### Synthesis of compound [43]

Four point five grams of an orange powder (yield 54%) was obtained by synthesizing according to the same method as in Synthesis Example 1, except that 3-phenyl-5-phenylbenzothiophene was used as an alternative to 3-phenylbenzofuran. The ¹H-NMR analysis result of the obtained powder was as shown below, and it was confirmed that the obtained orange powder was compound [43]. ¹H-NMR (CDCl₃ (d=ppm)): 7.32-8.01(m, 34H), 8.45(s, 2H).

Meanwhile, the compound [43] was sublimed and purified at a pressure of 1 x 10⁻³ and at approx. 290°C using an oil diffusion pump, to be used as a light-emitting element material. HPLC purity (area % at a measurement wavelength of 254 nm) was 99.2% before the sublimation purification and 99.3% after the sublimation purification.

### Synthesis Example 9

### Synthesis of compound [62]

Three point five grams of an orange powder (yield 52%) was obtained by synthesizing according to the same method as in Synthesis Example 1, except that N-methyl-3-phenylindole was used as an alternative to 3-phenylbenzofuran. The ¹H-NMR analysis result of the obtained powder was as shown below, and it was confirmed that the obtained orange powder was compound [62].
¹H-NMR (CDCl₃ (d=ppm)): 3.60(s, 6H), 6.81-8.05(m, 26H), 8.41(s, 2H).

Meanwhile, the compound [62] was sublimed and purified at a pressure of 1 x 10⁻³ and at approx. 240°C using an oil diffusion pump, to be used as a light-emitting element material. HPLC purity (area % at a measurement wavelength of 254 nm) was 99.0% before the sublimation purification and 99.1% after the sublimation purification.

### Synthesis Example 10

### Synthesis of compound [94]

The method described in JP 2005-53900 A was used for synthesis. Four point seven grams of 2- (2-methoxybenzoyl) -3, 5-bis (4-t-butylphenyl)pyrrole and 3. 3 g of 2,4-bis(4-t-butylphenyl)pyrrole were dissolved into 30 mL of 1,2-dichloroethane, and 1.5 g of phosphorus oxychloride was added, the mixture being heated under reflux for 12 hours to perform reaction. The reaction mixture was cooled to room temperature, and 5.2 g of diisopropylethylamine and 5.6 g of boron trifluoride diethyl ether complex were added, and the mixture was stirred for 6 hours. Fifty milliliters of water was added and dichloromethane was added, the organic layer being extracted and concentrated. The crude product was purified by silica gel chromatography, to obtain 4.5 g of compound [94].

### Synthesis Example 11

### Synthesis of compound [93]

The method described in JP 2005-53900 A was used for synthesis. Four point seven grams of 2-(4-methoxybenzoyl)-3,5-bis(4-t-butylphenyl)pyrrole and 3.3 g of 2,4-bis(4-t-butylphenyl)pyrrole were dissolved into 30 mL of 1,2-dichloroethane, and 1.5 g of phosphorus oxychloride was added, the mixture being heated under reflux for 12 hours to perform reaction. The reaction mixture was cooled to room temperature, and 5.2 g of diisopropylethylamine and 5.6 g of boron trifluoride diethyl ether complex were added, the mixture being stirred for 6 hours. Fifty milliliters of water was added and dichloromethane was added, the organic layer being extracted and concentrated. The crude product was purified by silica gel chromatography, to obtain 5.0 g of compound [93].

### Working Example 1

A light-emitting element using compound [7] and compound [94] was produced as described below. On a 30 x 40 mm glass substrate (electron beam vapor deposition product, 15 Ω/□, produced by Asahi -Glass Co., Ltd.), an ITO electroconductive film-with- a thickness of 150 nm and a size of 30 x 13 mm was formed in the central portion of the glass substrate as an anode. The substrate with the anode formed thereon was ultrasonically washed using "Semicoclean 56" (trade name, produced by Furuuchi Chemical Corp.) for fifteen minutes, and washed with ultrapure water. The substrate was treated with UV-ozone for 1 hour immediately before the element was produced, and installed in a vacuum evaporation apparatus that was evacuated to a vacuum degree of 5 x 10⁻⁴ Pa or less. A resistance heating method was used to vapor-deposit copper phthalocyanine as a hole injection material by 10 nm, and to vapor-deposit 4,4'-bis(N-(1-naphthyl)-N-phenylamino)biphenyl as a hole transport material by 50 nm. Then, as light-emitting materials, compound [7] as a host material and compound [94] (fluorescence peak wavelength in the state of 1.0 x 10⁻⁶ mol/L dichloromethane solution: 612 nm) as a dopant material were vapor-deposited with a thickness of 40 nm to achieve a dopant concentration of 0.5 wt%. Subsequently the following E-1 was laminated with a thickness of 35 nm as an electron transport material. Then, lithium fluoride was vapor-deposited by 0.5 nm and subsequently aluminum was vapor-deposited by 1,000 nm as a cathode, to produce a 5 x 5 mm square element. The film thickness stated here is a value displayed by a crystal oscillator film thickness monitor. From the light-emitting element, highly efficient red light emission with a light emission efficiency of 4.51 m/W could be obtained. The light-emitting element was DC-driven at 40 mA/cm², and the luminance half-life was 3,500 hours.

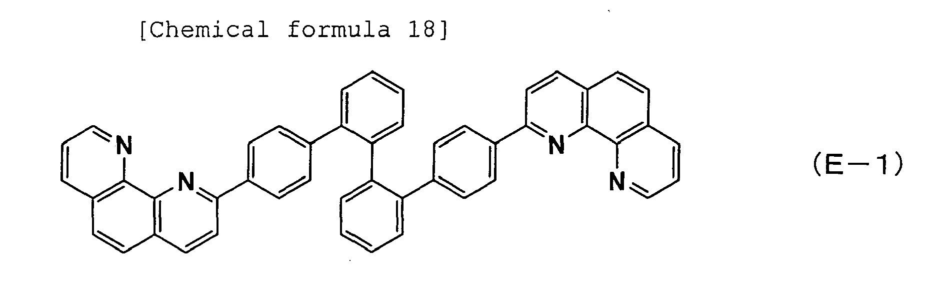

### Comparative Example 1

A light-emitting element was produced as described in Working Example 1, except that 1,4-diketo-2,5-bis(3,5-dimethylbenzyl)-3,6-bis(4-methylphenyl) pyrrole[3,4-c]pyrrole (hereinafter referred to as H-1) was used as the host material. From this element, red light emission with a light emission efficiency of 2.51 m/W could be obtained. When the light-emitting element was DC-driven at 40 mA/cm², luminance half-life was 600 hours. Meanwhile, as the H-1, the compound synthesized according to the example of EP 0133156 was used.

### Comparative Example 2

A light-emitting element was produced as described in Working Example 1, except that the following H-2 was used as the host material. From this element, pink light emission with a light emission efficiency of 2.21 m/W could be obtained. The light-emitting element was DC-driven at 40 mA/cm², and luminance half-life was 400 hours. Meanwhile, as the H-2, a commercial available product (produced by Tokyo Chemical Industry Co., Ltd.) was used.

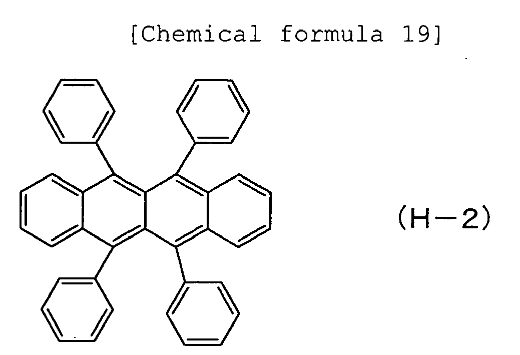

### Comparative Example 3

A light-emitting element was produced as described in Working Example 1, except that the following H-3 was used as the host material. From the element, pink light emission with a light emission efficiency of 3.01 m/W could be obtained. The light-emitting element was DC-driven at 40 mA/cm², and luminance half-life was 900 hours. Meanwhile, as the H-3, the compound synthesized according to Production Example 1 of WO 2007/097178 was used.

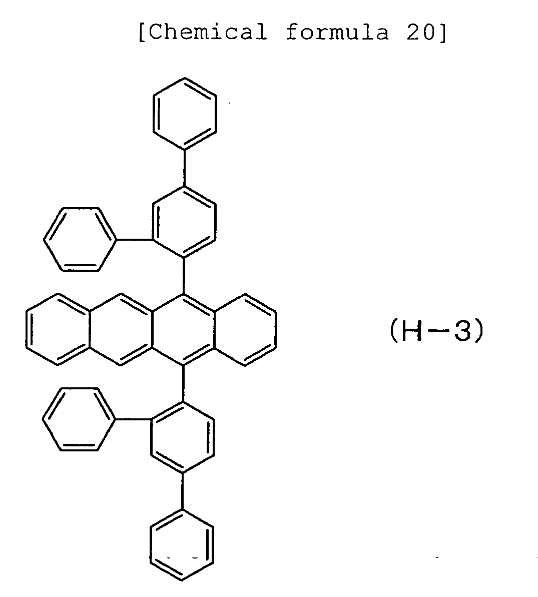

### Working Examples 2 to 19

Light-emitting elements were produced as described in Working Example 1, except that the host materials, dopant materials and electron transport materials shown in Table 1 were used. Meanwhile, as the compound [64] of Working Example 15, a commercially available product (Pyrromethene 546 produced by Exciton, USA) was used.

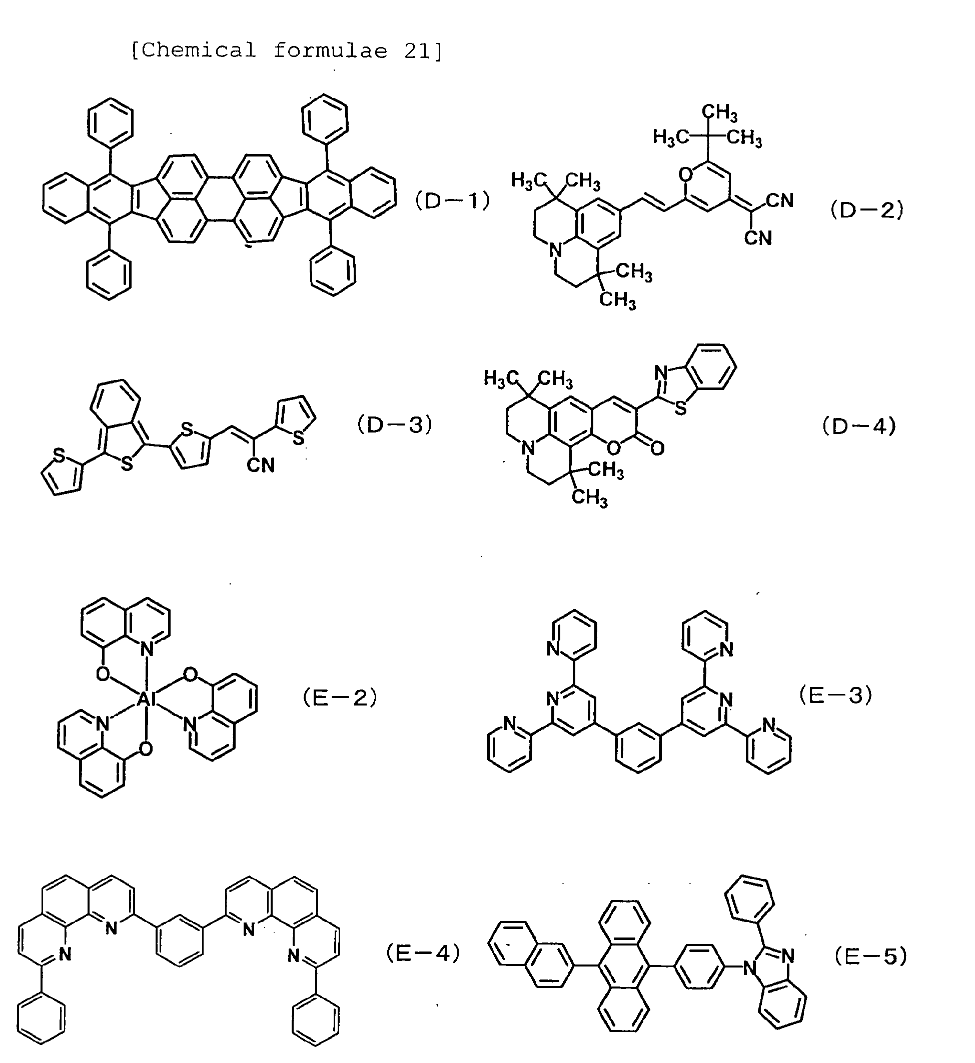

### Comparative Examples 4 and 5

Light-emitting elements were produced as described in Working Example 1, except that the host materials, dopant materials and electron transport materials shown in Table 1 were used.

**Table 1**

| | Light-emitting layer | | Electron transport material | Emitted light color | Light emission efficiency | Luminance half-life |
|---|---|---|---|---|---|---|
| | Host material | Dopant material (fluorescence peak wavelength in diluted solution) | | | | |
| Working Example 1 | Compound [7] | Compound [94] (612 nm) | E-1 | Red | 4.5 | 3500 |
| Comparative Example 1 | H-1 | Compound [94] (612 nm) | E-1 | Red | 2.5 | 600 |
| Comparative Example 2 | H-2 | Compound [94] (612 nm) | E-1 | Red | 2.2 | 400 |
| Comparative Example 3 | H-3 | Compound [94] (612 nm) | E-1 | Red | 3.0 | 900 |
| Working Example 2 | Compound [1] | Compound [94] (612 nm) | E-1 | Red | 3.2 | 2000 |
| Working Example 3 | Compound [6] | Compound [94] (612 nm) | E-1 | Red | 3.5 | 2500 |
| Working Example 4 | Compound [21] | Compound [94] (612 nm) | E-1 | Red | 4.2 | 3200 |
| Working Example 5 | Compound [43] | Compound [94] (612 nm) | E-1 | Red | 3.8 | 3000 |
| Working Example 6 | Compound [62] | Compound [94] (612 nm) | E-1 | Red | 3.5 | 2800 |
| Working Example 7 | Compound [16] | Compound [94] (612 nm) | E-1 | Red | 4.4 | 3400 |
| Working Example 8 | Compound [18] | Compound [94] (612 nm) | E-1 | Red | 4.2 | 3400 |
| Working Example 9 | Compound [22] | Compound [94] (612 nm) | E-1 | Red | 4.3 | 3300 |
| Working Example 10 | Compound [7] | Compound [93] (606 nm) | E-1 | Red | 4.3 | 3300 |
| Working Example 11 | Compound [7] | 0-1 (602 nm) | E-1 | Red | 3.1 | 1800 |
| Working Example 12 | Compound [7] | D-2 (602 nm) | E-1 | Red | 2.7 | 1400 |
| Comparative Example 4 | Compound [7] | D-3 (655 nm) | E-1 | Red | 2.2 | 1600 |
| Comparative Example 5 | Compound [7] | D-4 (506 nm) | E-1 | Green | 3.0 | 1200 |
| Working Example 13 | H-2 | D-1 (602 nm) | E-1 | Red | 2.0 | 300 |
| Working Example 14 | H-3 | D-1 (602 nm) | E-1 | Red | 2.5 | 700 |
| Working Example 15 | Compound [7] | Compound [64] (524 nm) | E-1 | Green | 3.3 | 2600 |
| Working Example 16 | Compound [7] | Compound [94] (612 nm) | E-2 | Red | 2.0 | 3000 |
| Working Example 17 | Compound [7] | Compound [94] (612 nm) | E-3 | Red | 3.0 | 3500 |
| Working Example 18 | Compound [7] | Compound [94] (612 nm) | E-4 | Red | 5.0 | 4000 |
| Working Example 19 | Compound [7] | Compound [94] (612 nm) | E-5 | Red | 4.6 | 3400 |

### INDUSTRIAL APPLICABILITY

The light-emitting element material of this invention can be used for light-emitting elements, and this invention can provide a light-emitting element material excellent in thin film stability. This invention can provide a light-emitting element with high light emission efficiency, excellent color purity and excellent durability. The light-emitting element of this invention can be used in such fields as display elements, flat panel displays, backlights, illumination, interior materials, signs, signboards, electrophotographic machines and light signal generators.

## Claims

1. A light-emitting element material comprising a naphthacene compound represented by general formula (1): 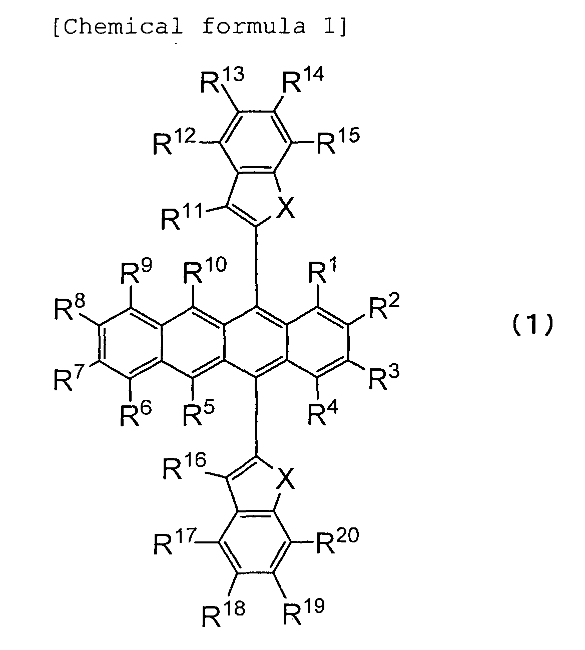 (R¹ to R²⁰ can be identical to or different from each other, and are selected from hydrogen, alkyl group, cycloalkyl group, heterocyclic ring group, alkenyl group, cycloalkenyl group, alkynyl group, alkoxy group, alkylthio group, aryl ether group, aryl thioether group, aryl group, heteroaryl group, halogen, carbonyl group, carboxyl group, oxycarbonyl group, carbamoyl group, amino group and silyl group; each pair of substituent groups adjacent to each other denoted by R¹² to R¹⁵ and R¹⁷ to R²⁰ can form a ring; X is selected from an oxygen atom, sulfur atom and -NR²¹-; and R²¹ is selected from hydrogen, alkyl group, cycloalkyl group, heterocyclic ring group, aryl group and heteroaryl group.)

2. A light-emitting element material, according to claim 1, wherein R¹¹ to R¹⁶ of the aforementioned general formula (1) denote respectively an aryl group or heteroaryl group.

3. A light-emitting element material, according to claim 1 or 2, wherein X of the aforementioned general formula (1) denotes an oxygen atom.

4. A light-emitting element capable of emitting light with electric energy, in which at least an organic layer exists between an anode and a cathode, wherein any one of the layers existing between the aforementioned anode and the aforementioned cathode contains the light-emitting element material set forth in any one of claims 1 to 3.

5. A light-emitting element, according to claim 4, wherein the organic layer contains a naphthacene compound represented by the general formula (1) and an organic fluorescent material with a fluorescence peak wavelength of 500 nm to 680 nm.

6. A light-emitting element, according to claim 5, wherein the organic fluorescent material with a fluorescence peak wavelength of 500 nm to 680 nm is a compound having a pyrromethene skeleton represented by general formula (2). 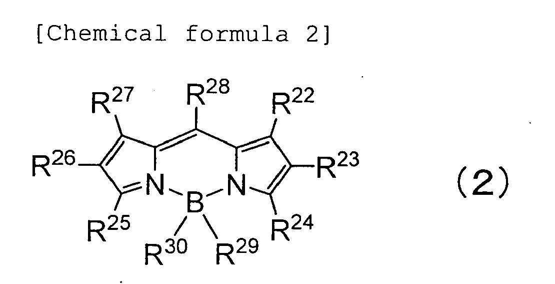 (R²² to R³⁰ can be identical to or different from each other and are selected from hydrogen, alkyl group, cycloalkyl group, heterocyclic ring group, alkenyl group, cycloalkenyl group, alkynyl group, alkoxy group, alkylthio group, aryl ether group, aryl thioether group, aryl group, heteroaryl group, cyano group, amino group, silyl group, -P(=O)R³¹R³² and a ring structure formed with an adjacent substituent group; R³¹ and R³² are respectively selected from an aryl group and heteroaryl group; R²⁹ and R³⁰ can be identical to each other or different from each other and are respectively selected from a halogen, hydrogen, alkyl group, aryl group and heterocyclic ring group.)

7. A light-emitting element, according to any one of claims 4 to 6, wherein at least an electron transport layer exists further between the organic layer and the cathode and contains a compound composed of a heteroaryl ring having an electron-acceptable nitrogen; and the compound composed of a heteroaryl ring is composed of one or more elements selected from carbon, hydrogen, nitrogen, oxygen, silicon and phosphorus.
